# EUROPEAN PATENT SPECIFICATION

(11) **EP 3 052 480 B1**
(45) Date of publication and mention of the grant of the patent: **11.11.2020**
(21) Application number: 14848887.7
(22) Date of filing: 29.09.2014
(51) Int. Cl.: A61K 31/427, C07D 277/46, C07D 417/04, A61K 31/4155, A61K 31/496, A61K 31/498, A61K 31/635, A61K 31/70, A61K 31/7034, A61K 31/7056, G01N 33/566, A61K 31/192, A61K 31/63, A61P 29/00

(54) **COMPOSITIONS ASSOCIATED WITH AND METHODS OF MANAGING NEUTROPHIL MOVEMENT**
ZUSAMMENSETZUNGEN UND VERFAHREN ZUR VERWALTUNG NEUTROPHILER BEWEGUNGEN
COMPOSITIONS ASSOCIÉES ET PROCÉDÉS DE GESTION DE DÉPLACEMENT DE NEUTROPHILES

(30) Priority: 30.09.2013 US 201361884395 P
(43) Date of publication of application: 10.08.2016
(73) Proprietor: THE TEXAS A&M UNIVERSITY SYSTEM, College Station, TX 77843-3369 (US)
(72) Inventor: GOMER, Richard H., College Station, TX 77845 (US); COX, Nehemiah, College Station, TX 77840 (US)
(74) Representative: Müller-Boré & Partner Patentanwälte PartG mbB
(86) International application number: PCT/US2014/058029
(87) International publication number: WO 2015/048633

(56) References cited:
- US-A1- 2009 029 998
- US-A1- 2009 048 209
- US-A1- 2010 242 124
- US-A1- 2013 039 897
- E. BLATTES ET AL: "Mannodendrimers prevent acute lung inflammation by inhibiting neutrophil recruitment", PROCEEDINGS NATIONAL ACADEMY OF SCIENCES PNAS, vol. 110, no. 22, 28 May 2013 (2013-05-28) , pages 8795-8800, XP055361247, US ISSN: 0027-8424, DOI: 10.1073/pnas.1221708110
- M. JACK BORROK ET AL: "Non-carbohydrate Inhibitors of the Lectin DC-SIGN", JOURNAL OF THE AMERICAN CHEMICAL SOCIETY, vol. 129, no. 42, 1 October 2007 (2007-10-01), pages 12780-12785, XP055336275, ISSN: 0002-7863, DOI: 10.1021/ja072944v
- SHANE L. MANGOLD ET AL: "Quinoxalinoneinhibitors of the lectin DC-SIGN", CHEMICAL SCIENCE, vol. 3, no. 3, 1 January 2012 (2012-01-01) , pages 772-777, XP055361358, United Kingdom ISSN: 2041-6520, DOI: 10.1039/C2SC00767C
- MANUEL ANDREINI ET AL: "Second generation of fucose-based DC-SIGN ligands : affinity improvement and specificity versus Langerin", ORGANIC & BIOMOLECULAR CHEMISTRY, vol. 9, no. 16, 1 January 2011 (2011-01-01), page 5778, XP055361373, GB ISSN: 1477-0520, DOI: 10.1039/c1ob05573a

## Description

The present invention relates to CD209-binding compounds or antibodies for use in methods of treating an acute injury or a chronic or long-term disease characterized by an excess of neutrophils in a body region by reducing the number of neutrophils in said body region.

### Glycosylation

The regulation of the immune system by proteins often depends on the glycosylation of those proteins. Glycosylation refers to the attachment of carbohydrate molecules to proteins. Glycosylation is typically triggered by certain amino acids on the proteins. Cells can create diverse patterns of protein glycosylation to convey different messages. This phenomenon can be observed in antibodies (gamma globulins). Antibodies with fully processed N-linked glycans terminating in sialic acid bind macrophages and dendritic cells to up-regulate Interleukin 33 in the immune system. This ultimately leads to decreased sensitivity of macrophages to proinflammatory signals. In contrast, antibodies lacking terminal sialic acid bind immune cells to initiate an immune response.

Glycosylation, in addition to providing a plethora of conveyable messages, also acts as an identification key because the glycosylation patterns used by cells can be species specific. This uniqueness allows the immune system to recognize foreign and sometimes dangerous proteins or pathogens and to initiate an immune response.

### Glycosylation Receptors

Many cells in the human body have receptors on their surfaces that detect glycosylated proteins. These receptors are called lectin receptors and are important in receiving messages conveyed by glycosylation and in recognizing glycosylation patterns.

CD209 (also known as Dendritic Cell-Specific Intercellular adhesion molecule-3-Grabbing Non-integrin (DC-SIGN)) is a lectin receptor that uses calcium as a co-factor to bind glycosylated proteins. CD209 can be found on the surface of monocytes, macrophages, dendritic cells, and other cells, and binds mannose carbohydrates on pathogens and partially glycosylated proteins to initiate an immune response. This receptor also binds to fucose carbohydrates on the surface of fully processed glycosylated proteins to prevent the immune system from attacking the host.

Recently, CD209 was also recognized as a receptor for immuno-suppressive gamma globulins containing α(2,6)-glycosidic linkages of sialic acid as the terminal carbohydrate on their surfaces. This specific type of glycosylation binds CD209 to up-regulate the secretion of the cytokine, Interleukin 33. The end result of this up-regulation is decreased sensitivity of macrophages to proinflammatory molecules that signal through Fc gamma receptors.

### Tumor Necrosis Factor-Alpha (TNF-α)

TNF-α is a trimeric extracellular protein. It functions as a pleiotropic inflammatory cytokine. Most organs of the body appear to be affected by TNF-α, and the cytokine serves a variety of functions, many of which are not yet fully understood. It possesses both growth stimulating properties and growth inhibitory processes. TNF-α is produced by several types of cells, but especially by macrophages.

TNF-α plays an important role in the inflammatory response. It functions as an acute phase protein which initiates a cascade of cytokines and increases vascular permeability, thereby recruiting macrophages and neutrophils to a site of injury or infection. TNF-α secreted by macrophage causes blood clotting. TNF-α also encourages neutrophil migration and increases the adherence of neutrophils to a variety of extracellular matrices.

### Movement of Neutrophils From the Blood into Body Regions

Infections or injuries to tissues such as the lungs cause the damaged cells to recruit immune cells, including neutrophils and monocytes, to the injury site. The transmigration of neutrophils to the site of injury or infection requires the interaction of neutrophils with endothelial cells and extracellular matrices. In blood vessels, neutrophils are generally quiescent, but after an injury or infection, neutrophils begin to tether and roll on the blood vessel using the selectin family of adhesion molecules such as CD62L, CD62P, and P-selectin glycoprotein ligand-1 (PSGL-1). These adhesion molecules interact with endothelial cell adhesion molecules such as E-selectin, P-selectin, and PSGL-1. Activated endothelial cells also interact with neutrophil glycoproteins such as CD44 and CD43 through E-selectin to slow neutrophil rolling. CD44 interacts with E-selectin and causes the redistribution of PSGL-1 or L-selectin on rolling neutrophils, which then promotes the tethering of neutrophils and slows down the rolling velocity. The slow neutrophil rolling allows neutrophils to sense signals such as IL-8, TNF-α, GM-CSF, or fMLP from damaged cells or infection, and activate integrin adhesion molecules such as CD1b and CD18. IL-8 is a neutrophil chemoattractant that can induce neutrophil degranulation and enhance neutrophil production of reactive oxygen species. TNF-α and GM-CSF increase neutrophil adherence, release of reactive oxygen species, and phagocytosis. fMLP resembles bacterial waste products, and activates neutrophil chemotaxis.

The upregulation of the adhesion molecules CD11b and CD18 let neutrophils interact with endothelial ligands such as intercellular adhesion molecule-1 (ICAM-1), which causes neutrophils to firmly adhere to the endothelium and move through the blood vessel into an injured site. Integrin molecules such as CD11b and CD18 can also bind to extracellular matrix components such as fibronectin, fibrinogen, laminin, and collagen, and this binding aids in the movement of neutrophils through extracellular matrices. Other integrin adhesion molecules such as CD61 facilitate leukocyte migration, but little is known about their roles in neutrophil migration. Once activated neutrophils are at injured sites, they can release reactive oxygen species and proteases, and then engulf bacteria and debris by phagocytosis.

In the normal resolution of wound healing, activated neutrophils undergo programmed cell death, which prevents the release of reactive oxygen species from the neutrophils, thereby preventing any cell damage in the surrounding tissue. Because activated neutrophils can damage surrounding cells, cytokines such as IL-4 and IL-10 inhibit excessive recruitment of neutrophils into the site of injury. IL-4 and IL-10 inhibit the production of IL-8 and the release of TNF-α and IL-1β, which in turn limits granulocyte (including neutrophil) accumulation and activation. Lipid mediators such as lipoxin A4 (LXA4) and lipoxin B4 (LXB4) inhibit neutrophil recruitment by reducing neutrophil adhesion to endothelial cells and vascular permeability. Other lipid mediators including D- and E-series resolvins and protectins also inhibit transendothelial migration of neutrophils.

### Effects on Neutrophils

Neutrophils are immune system cells found in the blood and elsewhere that are involved in the early stages of immune response and inflammation. Neutrophils are typically some of the first cells to leave the blood stream and enter the site of an injury or infection. Neutrophils begin to arrive at an infection or injury site within seconds to minutes, depending on blood circulation to the region. Once there, they release chemicals that further increase the immune or inflammatory response, for instance by recruiting more immune systems cells.

Serious medical conditions can result in patients who have low numbers of neutrophils in their blood stream or whose neutrophils do not appropriately leave the blood stream when needed. Such problems can occur due to genetic disorders, diseases, such as immunological disease, or the effects of immunosuppressant medications. These patients may be unable to respond appropriately to infection or injury, preventing proper wound healing or allowing infection to set in and become harder to combat later. Accordingly, a need exists for a method of increasing the ability of neutrophils to leave the blood stream and enter injury or infection sites in such patients.

Localized increases in the number of neutrophils in otherwise normal patients may also sometimes be useful. For instance, neutrophils may play a role in combating cancer. Cancer patients with otherwise normal neutrophil levels and activity might benefit from increased influx of neutrophils to a cancerous tumor or lesion, to the surrounding area, or even to the entire affected organ to help prevent metastasis. Currently, neutrophil-specific treatments are unavailable.

Serious medical conditions may also result when too many neutrophils enter a location in the body. Because the neutrophils have the ability to recruit additional immune system cells and to otherwise enhance the immune and inflammatory responses, they may cause harmful inflammation or damage from the immune system. There are two primary reasons for this to occur, either an immunological disorder or a severe injury.

An overabundance of neutrophils may result from an immunological disorder in any number of ways. For instance, some patients may simply produce too many neutrophils or may produce neutrophils that leave the blood stream too easily. Such patients may be at risk for an over-abundance of neutrophils in even a minor injury. More commonly, the patient may have an autoimmune disease, such as rheumatoid arthritis. Unusually high numbers of neutrophils are found in the joints of patients with rheumatoid arthritis, indicating their role in the development and progression of that disease. Unusually high and harmful numbers of neutrophils may be found in any organ or tissue affected by an autoimmune disease.

Another cause for a harmful overabundance of neutrophils is a severe injury. The body is often not able to address severe injuries and actually causes more harm in attempting to do so. For instance, severe injuries often result in a runaway effect, in which the body responds at higher and higher levels and in more and more ways until the negative effects of the over-response outweigh any positive ones.

For instance, there are over 200,000 cases of acute respiratory distress syndrome (ARDS) in the United States each year. ARDS can result from any severe injury to the lungs, such as infection or inhaling acidic materials, such as vomitus, or from excess smoke inhalation, such as during house fires. During ARDS, a large number of lung cells are damaged, causing a rapid influx of neutrophils from the blood stream into the lungs. Once there, the neutrophils release reactive oxygen species and proteases that cause still further damage to lung cells, including the remaining healthy lung cells. These additional damages cause more neutrophils to enter the lungs, resulting in still more damage. Current treatments for ARDS are ineffective at halting this cycle of neutrophil influx and lung damage. As a result, 40% of all patients who develop ARDS die shortly thereafter.

Many ARDS patients do not sustain fatal levels of lung damage during the initial onslaught. As a result, if there were a treatment that, within a matter of a few hours, could deter neutrophils from entering the damaged lung, these patients could be saved.

Neutrophils also play a role in chronic obstructive pulmonary disease (COPD). Even though this disease is chronic, rather than acute in nature, there appears to be a constant influx of unhealthy levels of neutrophils into the lung tissue of COPD patients. Furthermore, the amount of neutrophils in the lungs correlates with the severity of the disease. Thus, if the number of neutrophils present in the lungs of COPD patients could be reduced, a corresponding improvement in symptoms is expected.

Treatments able to affect neutrophils entering the lung might also be useful in other situations. For instance, they may be able to help prevent any damaging effects of minor lung injuries, such as inflammation caused by minor air pollution.

Finally, treatments able to deter neutrophils from entering other inappropriate regions or able to drive them from those regions, such as arthritic joints, may be useful in treating other diseases, like rheumatoid arthritis

Other problems may result from too few neutrophils in a region of the body. The problems may be corrected or ameliorated by encouraging neutrophil influx.

In this context, Blattes *et al.* (Blattes, E. et al.; PNAS, 110(22); 2003; pp. 8795-8800) describes compounds preventing acute lung inflammation by inhibiting neutrophil recruitment.

The present invention relates to the following items:
1. A CD209-binding compound or antibody for use in a method of treating an acute injury or a chronic or long-term disease characterized by an excess of neutrophils in a body region by reducing the number of neutrophils in said body region, wherein the compound or antibody is administered within 24 hours, within 48 hours, or within 72 hours after an event triggering neutrophil entry into the body region.
2. The compound or antibody for use according to item 1, wherein the compound is Compound 1 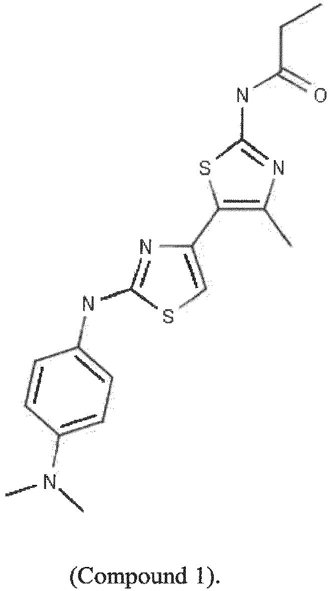
3. The compound or antibody for use of item 1, wherein the compound is a compound of formula I 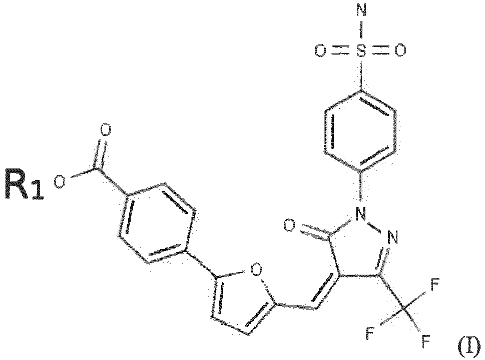 in which
   R₁ is an aryl group, an alkyl group, a substituent with both aryl and alkyl components, or H.
4. The compound or antibody for use of item 3, wherein the compound is Compound 2 or Compound 3 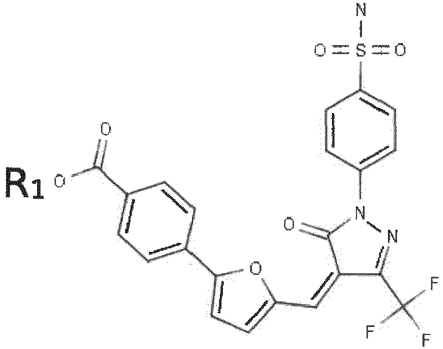 (Compound 2, wherein R₁ is a propyl group)
   (Compound 3, wherein R₁ is a methyl group).
5. The compound or antibody for use of item 1, wherein the compound is a compound of formula II 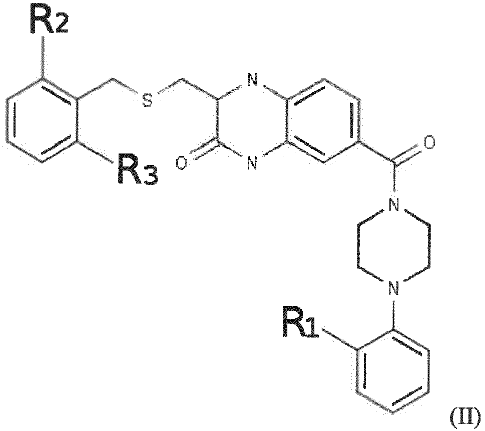 wherein
   R₁ is an aryl group, an alkyl group, a substituent with both aryl and alkyl components, or H, and
   R₂ and R₃ both are a monovalent halide, an aryl, an alkyl, a substituent with both aryl or alkyl components, or H.
6. The compound or antibody for use of item 1, wherein the compound is a compound of formula III 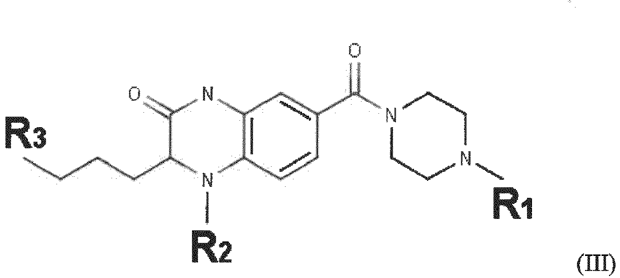 wherein
   R₁, R₂, and R₃ each are an alkyl group, an aryl group, a substituent with both aryl and alkyl components, or H.
7. The compound or antibody for use of item 6, wherein R₁ and R₃ both are an aryl group.
8. The compound or antibody for use of item 1, wherein the compound is a compound of formula IV 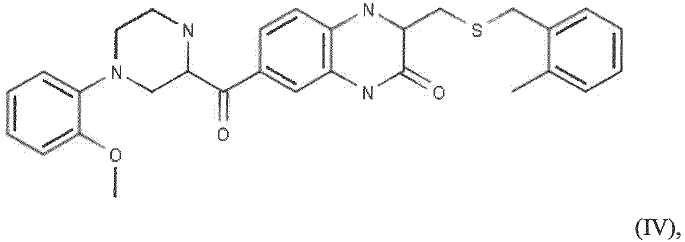 or a compound of formula V 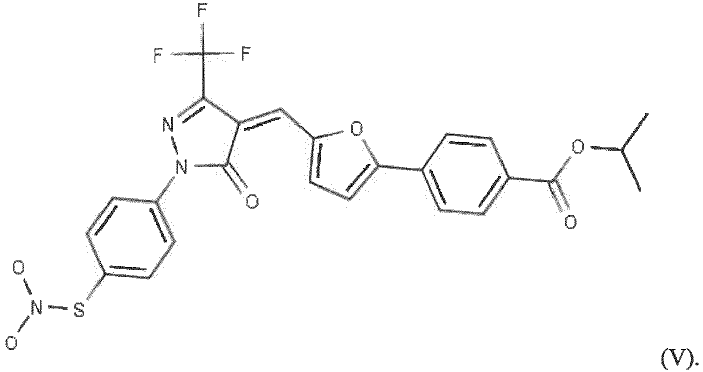
9. The compound or antibody for use of item 1, wherein the compound is a compound of formula VI 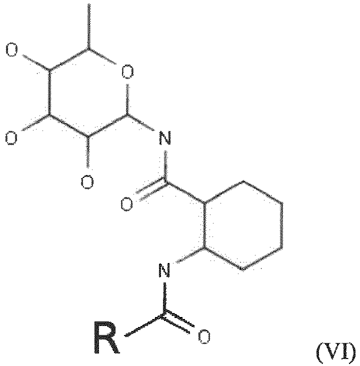 wherein
   R is an aryl group, an alkyl group, a substituent with both aryl and alkyl components, or H.
10. The compound or antibody for use of item 1, wherein the antibody is an anti-human CD209 antibody.
11. The compound or antibody for use according to item 1, wherein the body region is the lungs and the compound or antibody is administered by inhalation or by nebulizer.
12. The compound or antibody for use according to item 1, wherein the compound or antibody is administered by injection, topical administration, oral administration, or systemic administration.

This disclosure is based upon the finding of a new activity of CD209-binding compounds as inhibitors of neutrophil adhesion. Various aspects of the disclosure may be carried out using a composition containing a CD209-binding compound or an anti-CD209 antibody.

One aspect of the present disclosure is a method of reducing the number of neutrophils in a body region including by administering a CD209-binding compound or antibody in an amount and for a time sufficient to suppress neutrophil movement into the body region.

Another aspect of the present disclosure is a method of reducing the number of neutrophils in the lungs of a patient suffering from acute respiratory distress syndrome (ARDS) including by administering a CD209-binding compound or antibody to the patient in an amount and for a time sufficient to suppress neutrophil movement into the lungs.

An alternative aspect of the present disclosure is a method of reducing the number of neutrophils in the lungs of a patient suffering from acute lung injury (ALI) including by administering a CD209-binding compound or antibody to the patient in an amount and for a time sufficient to suppress neutrophil movement into the lungs.

An additional aspect of the present disclosure is a method of treating a disease, wherein at least one of the indicators of the disease is the number of neutrophils in a body region of a patient, including by administering a CD209-binding compound or antibody to the patient in an amount and for a time sufficient to suppress neutrophil movement into the body region.

Elements from all four aspects in the four preceding paragraphs may be combined with one another. In addition, elements of all four aspects in the four preceding paragraph may be combined with any of the following elements, which may also be combined with one another, where appropriate. A. The disease may be further defined as including neutrophil-mediated tissue damage. B. The disease may affect the lungs. C. The disease may affect the liver. D. The disease may include alcohol-induced neutrophilic steatohepatitis or acetaminophen overdose. E. The disease may include acute phase tissue transplant rejection. F. The disease may include traumatic brain injury. G. The disease may affect the kidneys. H. The disease may include acute glomerulonephritis and/or renal inflammation. I. The disease may affect the bowels. J. The disease may include postoperative ileus. K. The disease may affect the skin. L. The disease may include Sweet's syndrome/acute febrile neutrophilic dermatosis, rheumatoid neutrophilic dermatitis, pyoderma gangrenosum, subcorneal pustular dermatosis, Behcet's syndrome, palmoplantar pustulosis, neutrophilic eccrine hidradenitis, bowel-associated dermatosis-arthritis syndrome, or synovitis-acne-pustulosis-hyperostosis osteomyelitis (SAPHO) syndrome. M. The disease may be systemic. N. The disease may include septic shock. O. The disease may include acute pancreatitis. P. The CD209-binding compound or antibody may be administered a short time after an event triggering neutrophil entry into the lungs. Q. The short time may be within 30 minutes, within 45 minutes, or within 60 minutes. R. The short time may be within 2 hours, within 4 hours, within 6 hours, or within 8 hours. S. The short time may be within 24 hours, within 48 hours, or within 72 hours. T. The compound may include Compound 1. U. The compound may include a compound of formula I in which R₁ includes an aryl group, an alkyl group, a substituent with both aryl and alkyl components, or H. V. The compound may include Compound 2. W. The compound may include Compound 3. X. The compound may indlude a compound of formula II, wherein R₁ comprises an aryl group, an alkyl group, a substituent with both aryl and alkyl components, or H, and R₂ and R₃ both include a monovalent halide, an aryl, an alkyl, a substituent with both aryl or alkyl components, or H. Y. The compound may include a compound of formula III, wherein R₁, R₂, and R₃ each include an alkyl group, an aryl group, a substituent with both aryl and alkyl components, or H. Z. The compound may be the compound of Y wherein R₁ and R₃ both comprise an aryl group. AA. The compound may include a compound of formula IV or its derivatives. BB. The compound may include a compound of formula V and/or its derivatives. CC. The compound may include a compound of formula VI, wherein R includes an aryl group, an alkyl group, a substituent with both aryl and alkyl components, or H. DD. The compound may include a compound of formula VII, wherein R includes an aryl group, an alkyl group, a substituent with both aryl and alkyl components, or H. EE. The compound may include Compound 24. FF. The compound may include Compound 25. GG. The compound may include Compound 26. HH. The compound may include Compound 27. II. The antibody may include an anti-human CD209 antibody. JJ. The anti-human CD209 antibody may include clone 1B10, clone eB-h209, clone ab89186, and clone ERP5588. KK. Antibodies may include monoclonal antibodies, polyclonal antibodies, antibody fragments, human antibodies, humanized antibodies, chimeric antibodies, bispecific antibodies, recombinant antibodies, IgG1 antobodies, IgG2 antibodies, IgG3 antibodies, and IgG4 antibodies. LL. Antibodies may include antibodies that bind to the same epitopes as clone 1B10, clone eB-h209, clone ab89186, or clone ERP5588. MM. The body region may include the lungs and administering may include administering by inhalation. NN. Administering by inhalation may include administering by nebulizer. OO. Administering may include injection. PP. Administering may include topical administration. QQ. Topical administration may include administration in the form of a cream, ointment, emollient, gel, foam, or transdermal patch. RR. Administering may include oral administration. SS. Administering may include systemic administration. TT. Administering a compound in an amount of 0.5 mg/kg of patient or target location or less. UU. Administering a compound in an amount of 0.1 mg/kg of patient or target location. VV. Administering a compound in an amount of between 0.001 and 0.05 mg/kg of patient or target location. WW. Administering a compound in an amount of between 0.01 and 0.1 mg/kg of patient or target location or less. XX. Administering an antibody at a concentration of 0.25 mg/mL or less. YY. Administering an antibody at a concentration of 0.125 mg/mL or less. ZZ. Administering an antibody at a concentration of between 0.01 mg/mL and 0.25 mg/mL. AAA. Administering an antibody at a concentration of between 0.01 mg/mL and 0.125 mg/mL.

The disclosure further includes a screening method including screening a compound or antibody to detect whether it binds CD209, then selecting a compound or antibody able to bind CD209 and further screening the compound or antibody to determine if it affects neutrophil movement or adhesion, reduces the number of neutrophils in a body region, or treats a disease affected by neutrophil movement. A compound or antibody thus selected may have any combination of elements of any of the two preceding paragraphs. Screening a compound or antibody to detect whether it bind CD209 may include high-throughput screening.

A more complete understanding of the present embodiments and advantages thereof may be acquired by referring to the following description taken in conjunction with the accompanying drawings, which depict embodiments of the present disclosure.

The figures show:
FIGURE 1A illustrates the normal adhesion of a neutrophil on a surface or in a tissue. Movement is indicated by dashed arrow.
FIGURE 1B illustrates the inhibitory effect a CD209-binding compound or antibody on the adhesion of a neutrophil on a surface or in a tissue. Lack of movement is indicated by lack of dashed arrow.
FIGURE 2 illustrates the results from an experiment to detect the effect of CD209-binding compounds on human neutrophil adhesion. Values are mean ± SEM for three independent experiments. * indicates p < 0.05 (t-test).
FIGURE 3 illustrates the effect of compound 1 on human neutrophil spreading on fibronectin-coated 96-well plates. Values are mean ± SEM for three independent experiments. ** indicates p < 0.01 (t-test).
FIGURE 4A illustrates the effect of anti-human CD209 antibodies on human neutrophil adhesion to human fibronectin. Subtype matched IgG was used as negative control for each tested antibody. Values are mean +/- SEM (n=2) of counts normalized to the no antibody TNF-α control. * indicates p < 0.05 (t-test).
FIGURE 4B illustrates the effect of anti-human CD209 antibodies clone eB-h209 and ab89186 on neutrophil adhesion to human fibronectin at different concentrations. Values are counts normalized to the no antibody TNF-α control.
FIGURE 5A illustrates the effect of compound 1 on neutrophils (Ly6G), inflammatory macrophages (CD11b), alveolar macrophages (CD11c), and leukocytes (CD45) in the bronchoalveolar lavage fluid from the lungs of C57BL/6 mice. Rat IgG1 indicates an isotype control antibody. Values are mean ± SEM, n=4. * indicates p<0.05 by 1-way ANOVA, Holm-Bonferroni test.
FIGURE 5B illustrates the effect of compound 1 on neutrophils (Ly6G), inflammatory macrophages (CD11b), alveolar macrophages (CD11c), CD206 positive cells, and leukocytes (CD45) in the lung sections of C57BL/6 mice. Rat IgG1 indicates an isotype control antibody. Values are mean ± SEM, n=4. * indicates p<0.05 by 1-way ANOVA, Holm-Bonferroni test.

The current disclosure relates to methods of regulating neutrophil movement in a patient or neutrophil numbers in a body region. In particular, it relates to methods of regulating neutrophil movement. According to one set of embodiments, neutrophil movement into body regions may be suppressed by administering a CD209-binding compound or antibody to the body. In an alternative embodiment, neutrophil movement into a body region may be suppressed in a localized manner by administering a CD209-binding compound or antibody in that region.

### CD209-Binding Compounds

CD209 is an extracellular protein found on the surface of monocytes and other immune system cells. CD209 binds to α(2,6)-glycoside linkages of sialic acids. Compounds able to bind CD209 may include both small molecules and biological molecules, such as antibodies.

In one embodiment, small molecule compounds may be based on mannose, mannose-containing oligosaccharides, or mannose mimetics. Mannose is a natural ligand for CD209.

In another embodiment, small molecule or biological molecule compounds may contain a mannose or mannose-like sugar epitope on a peptide, dendrimeric scaffold, or a polymer. Embodiments containing multiple epitopes may be capable of multivalent binding. Multivalent binding may lead to greater inhibition of fibrocyte formation.

In another embodiment, small molecule compounds may be based on fucose, fucose-containing oligosaccharides, or fucose mimics. Fucose is a natural ligand for CD209.

In another embodiment, small molecule or biological molecule compounds may contain a fucose or fucose-like sugar epitope on a peptide, dendrimeric scaffold, or a polymer. Embodiments containing multiple epitopes may be capable of multivalent binding. Multivalent binding may lead to greater inhibition of neutrophil adhesion.

In another embodiment, the compound may be a non-carbohydrate small molecule inhibitor. Such inhibitors may bind CD209 much more strongly than carbohydrate-based small molecules. In a more specific embodiment, such a compound may contain at least one amide carbonyl group, which may interact with a cation, particularly a divalent cation, such as Ca²⁺, when binding to CD209. In another more specific embodiment, such a compound may contain at least one aromatic group. In a further specific embodiment, the compound may contain both at least one amide carbonyl group and at least one aromatic group.

In one embodiment, the compound may have the chemical formula: 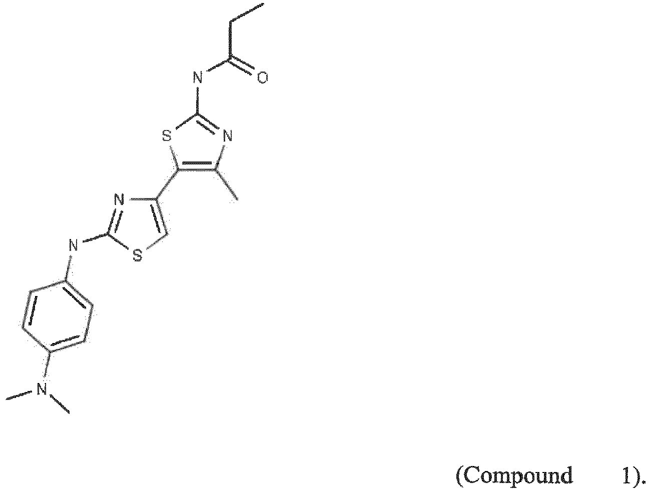 Compound 1 has an affinity for CD209 in which K_{d} = 1.6 +/- 0.5 µM.

In another embodiment, the compound may have the general chemical formula: 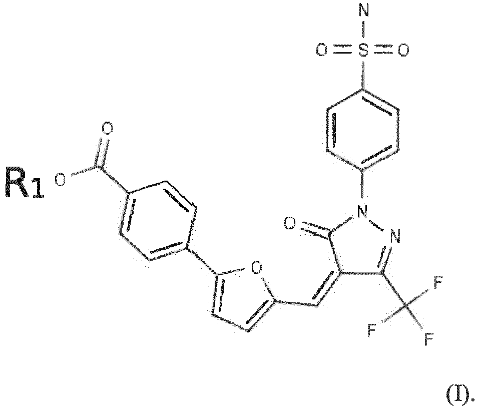 In more specific embodiments, R₁ may be a propyl (Pr) group (Compound 2). Compound 2 has an affinity for CD209 in which K_{d}=6.2 +/- 3.0 µM. In another specific embodiment, R₁ may be a methyl (Me) group (Compound 3). Compound 3 has an affinity for CD209 in which K_{d}=32 +/- 18 µM. In still other embodiments, R₁ may be an aryl group, an alkyl group, a substituent with both aryl and alkyl components, or H.

In another embodiment, the compound may have the general chemical formula: 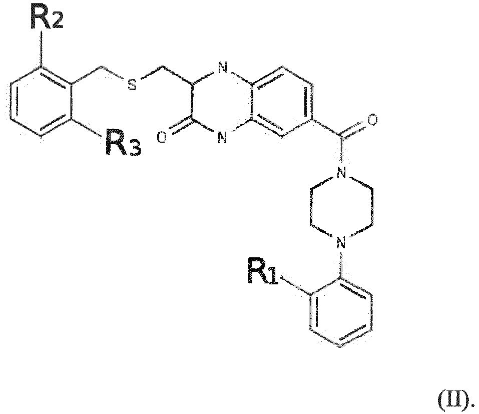 In more specific embodiments, R₁ may be an aryl group, an alkyl group, a substituent with both aryl and alkyl components, or H. R₂ and R₃ may be a monovalent halide, an aryl, an alkyl, a substituent with both aryl or alkyl components or H.

In another embodiment, the compound may have the general chemical formula: 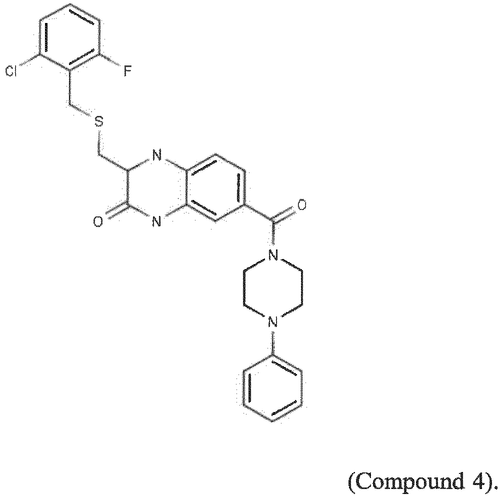 Compound 4 has an affinity for CD209 in which K_{d}=7.3 +/- 3.5 µM.

In another embodiment, the compound may have the general chemical formula: 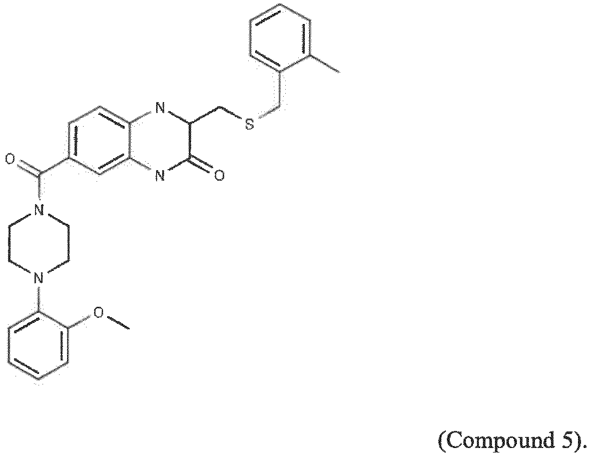 Compound 5 has an affinity for CD209 in which K_{d}=2.0 +/- 1.0 µM.

In another embodiment, the compound may have the general chemical formula: 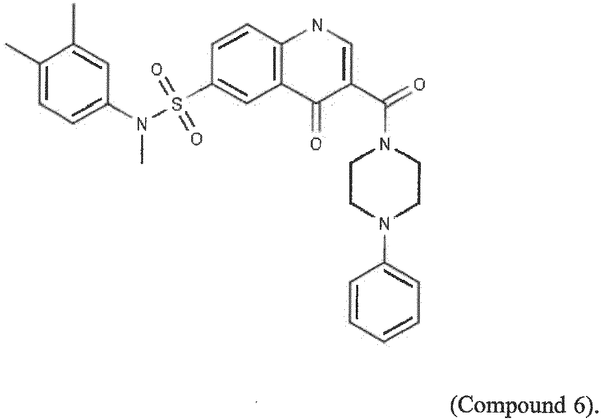 Compound 6 has an affinity for CD209 in which K_{d}=6.1+/- 3.0 µM.

In another embodiment, the compound may have the general chemical formula: 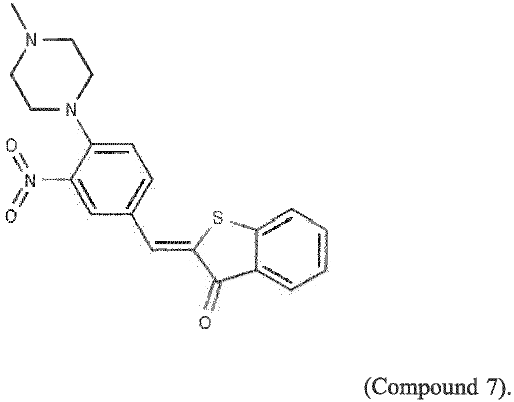 Compound 7 has an affinity for CD209 in which K_{d}=10.0+/- 9.0 µM.

In another embodiment, the compound may have the general chemical formula: 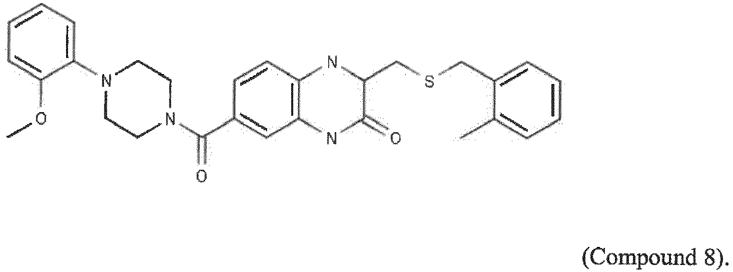 Compound 8 has an affinity for CD209 in which K_{d}=7.0 µM.

In another embodiment, the compound may have the general chemical formula: 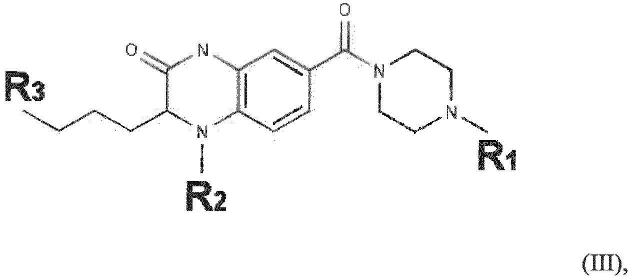 in which R₁, R₂ and R₃ are presented in TABLE 1 for various compounds.

**Table 1 - R groups for Formula III**

| **Compound** | **R₁** | **R₂** | **R₃** | **IC₅₀ (µM)*** |
|---|---|---|---|---|
| 9 | propyl (Pr) | ethyl (Et) | ethyl (Et) | 370+/-70.0 |
| 10 | ethyl (Et) | methyl (Me) | ethyl (Et) | 360 +/- 69.2 |
| 11 | methyl (Me) | methyl (Me) | methyl (Me) | 329 +/- 65.8 |
| 12 | methyl (Me) | H | methyl (Me) | 313 +/- 47.7 |
| 13 | tert-Butoxycarbonyl (Boc) | ethyl (Me) | methyl (Me) | 270+/-43.8 |
| 14 | 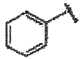 | benzyl (Bn) | 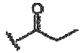 | 170 +/- 28.5 |
| 15 | 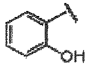 | H | 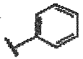 | 113 +/- 20.4 |
| 16 | 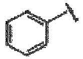 | H | 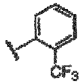 | 71 +/- 11 |
| 17 | 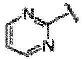 | ethyl (Et) | 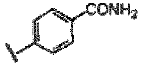 | 68 +/- 11 |
| 18 | 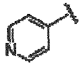 | benzyl (Bn) | 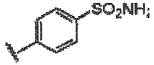 | 39 +/- 6.2 |
| 19 | 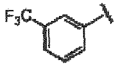 | H | 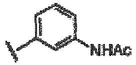 | 23 +/- 3.4 |
| 20 | 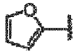 | H | 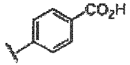 | 10 +/- 1.3 |
| 21 | 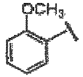 | H | 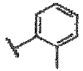 | 4.4 +/- 0.64 |
| 22 | 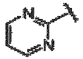 | H | 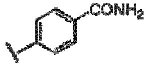 | **2.6** +/- 0.28 |
| 23 | 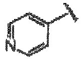 | H | 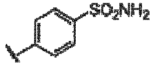 | 0.31 +/- 0.13 |

| | | | | |
|---|---|---|---|---|
| (*) values are reported as the concentration required to inhibit, by 50%, the binding of a fluorescently labeled mannosylated glycoconjugate to CD209 tetramer. | | | | |

Variations of each of the above compounds may additionally contain spacers, such as carbon chains, and in particular carbon chains containing between 2 and 50 carbon atoms, between the formula III molecule and the R₃ group, but only in as far as covered by the claims.

In more specific embodiments, the compound may have the general chemical formula of formula (III), in which R₁, R₂, and R₃ may each be an alkyl group, an aryl group, a substituent with both aryl and alkyl components, or H.

In further specific embodiments, the identity of R₁ may be more determinative of the ability to bind to CD209 than the identity of R₂ or R₃. R₁ may be a piperazine derivative functionalized with an aliphatic substituent, but for a more strongly binding compound, R₁ may contain an aryl group, particularly a nitrogen heterocycle.

In still further specific embodiments, R₂ may be H or another small substituent, with a decrease in CD209 binding observed with larger substituents.

In another specific embodiment, R₃ may contain an aryl group with a polar substituent in order to improve CD209 binding.

In a more specific embodiment, both R₁ and R₃ may contain an aryl group for improved CD209 binding.

In still other embodiments, the compound may contain or be derived from a quinoxalinone scaffold of the general chemical formula: 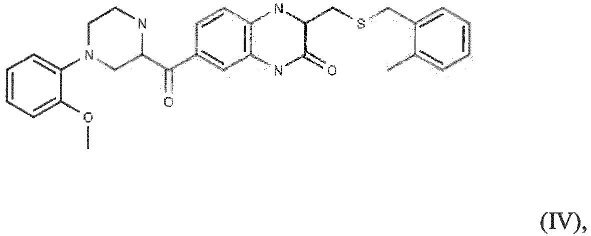 or a pyrazolone scaffold of the general chemical formula:

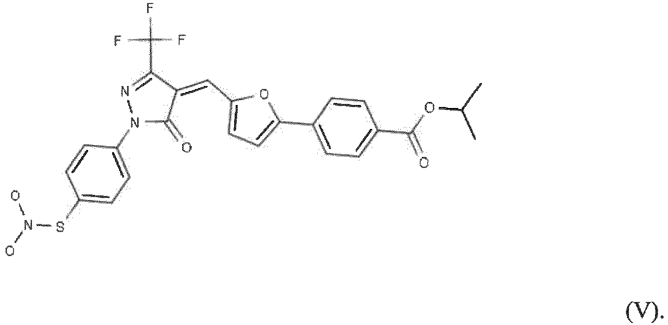

In another embodiment, the compound may have the general chemical formula:

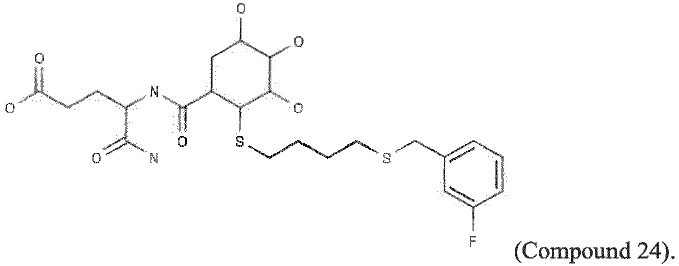

In another embodiment, the compound may have the general chemical formula:

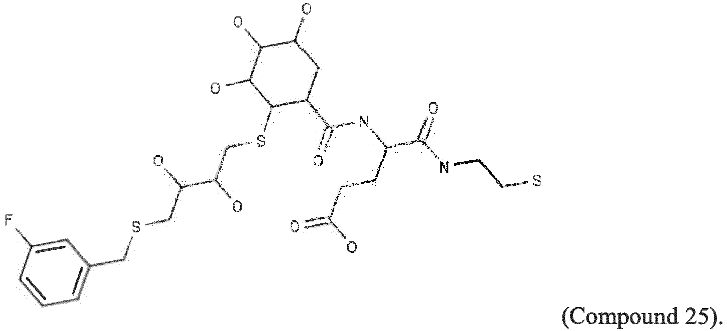

In still other embodiments, the compound may contain or may be conjugated to a carrier protein (*e.g.* Albumin) to form a glycoprotein ligand of CD209.

In still other embodiments, the compound may contain or be conjugated to a variety of scaffolds to form multivalent systems.

In another embodiment, the compound may include an artificial fuctose mimic with the general chemical formula:

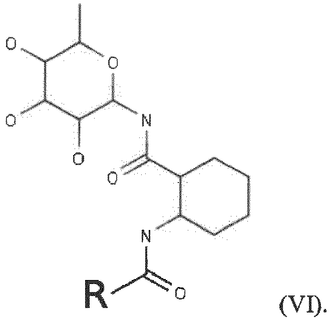

In more specific embodiments, the compound may have the general chemical formula of formula (VI), in which R may be an alkyl group, an aryl group, a substituent with both aryl and alkyl components, or H.

In further specific embodiments, the identity of R is determinative of the ability to bind to CD209.

In further specific embodiments, the *cis*-β- amino acid backbone of compound (VI) may determine the specificity and the ability of this compound to bind CD209.

In another aspect of the present disclosure (but not being part of the invention), the compound may have the general chemical formula:

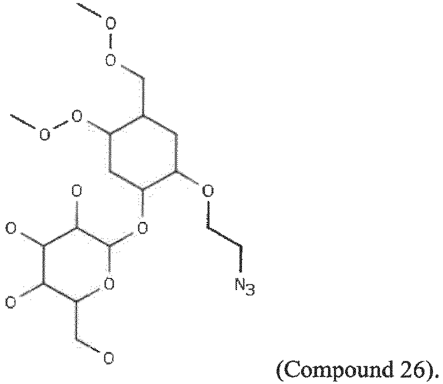

In another aspect of the present disclosure (not being part of the present invention), the compound may have the general chemical formula:

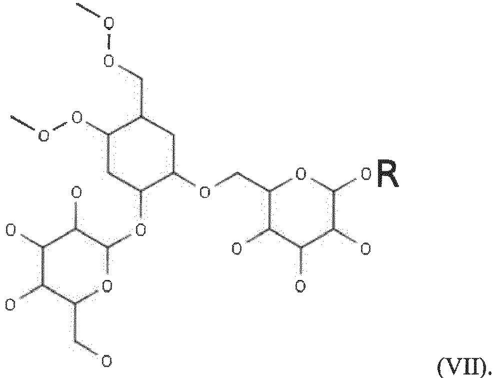

In more specific aspects , the compound may have the general chemical formula of formula (VII), in which R may be an alkyl group, an aryl group, a substituent with both aryl and alkyl components, or H.

In further specific aspects, the identity of R is determinative of the ability to bind to CD209.

In more specific aspects, the compound (VII) may be conjugated to Bolton-type dendrons to form multivalent structures.

In another embodiment, the compound may contain glactoside or mannoside in a polymeric form:

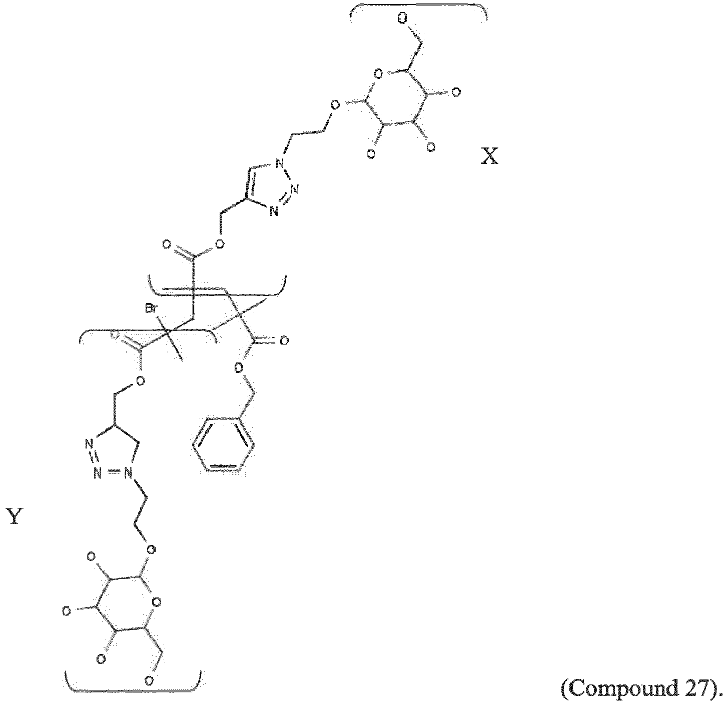

In more specific embodiments, compound 27 may have anywhere between 1 to hundreds of X or Y monomers to form a polymeric ligand of CD209.

In more specific embodiments, the number of X and Y monomers present in compound 27 determines its affinity for CD209.

CD209 plays a role in human immunodeficiency virus (HIV) infection, Ebola, Dengue fever, and Hepatitis C. Thus small molecules able to bind to CD209 and block binding of these pathogens to CD209 may also generally be used to prevent or treat fibrosing diseases.

Additional small molecules able to bind CD209 along with principles for designing additional such molecules and synthesis methods may be found in Borrak, M and Kiessling, L, Non-carbohydrate inhibitors of the lectin DC-SIGN, J. Am. Chem. Soc. 129: 12780-12785 (2007),Mangold, S et al., Quinoxalinone inhibitors of the lectin DC-SIGN, Chem. Sci. 3: 772-777 (2012), Sattin, S., A. Daghetti, M. Thépaut, A. Berzi, M. Sánchez-Navarro, G. Tabarani, J. Rojo, F. Fieschi, M. Clerici, and A. Bernardi. 2010. Inhibition of DC-SIGN-mediated HIV infection by a linear trimannoside mimic in a tetravalent presentation. ACS chemical biology 5: 301-12, Becer, C. R., M. I. Gibson, J. Geng, R. Ilyas, R. Wallis, D. a Mitchell, and D. M. Haddleton. 2010. High-affinity glycopolymer binding to human DC-SIGN and disruption of DC-SIGN interactions with HIV envelope glycoprotein. Journal of the American Chemical Society 132: 15130-2, Luczkowiak, J., S. Sattin, I. Sutkevičiutė, J. J. Reina, M. Sanchez-Navarro, M. Thépaut, L. Martinez-Prats, A. Daghetti, F. Fieschi, R. Delgado, A. Bernardi, and J. Rojo. 2011. Pseudosaccharide functionalized dendrimers as potent inhibitors of DC-SIGN dependent Ebola pseudotyped viral infection. Bioconjugate chemistry 22: 1354-65, Andreini, M., D. Doknic, I. Sutkeviciute, J. J. Reina, J. Duan, E. Chabrol, M. Thepaut, E. Moroni, F. Doro, L. Belvisi, J. Weiser, J. Rojo, F. Fieschi, and A. Bernardi. 2011. Second generation of fucose-based DC-SIGN ligands: affinity improvement and specificity versus Langerin. Organic & biomolecular chemistry 9: 5778-86,Prost, L. R., J. C. Grim, M. Tonelli, and L. L. Kiessling. 2012. Noncarbohydrate glycomimetics and glycoprotein surrogates as DC-SIGN antagonists and agonists. ACS chemical biology 7: 1603-8.

Anti-human CD209 antibodies may also be used to regulate neutrophil adhesion, recruitment, and activation. These antibodies include but are not limited to the anti-human clone 1B10 (Santa Cruz biotechnology, Dallas, TX), clone eB-h209 (eBioscience, San Diego, CA), clone ab89186 (Abcam, Cambridge, MA), and clone ERP5588 (Epitomics, Burlingame, CA). Antibodies may include antibodies that bind to the same epitopes as clone 1B10, clone eB-h209, clone ab89186, or clone ERP5588. Antibodies may include monoclonal antibodies, polyclonal antibodies, antibody fragments, human antibodies, humanized antibodies, chimeric antibodies, bispecific antibodies, recombinant antibodies, IgG1 antibodies, IgG2 antibodies, IgG3 antibodies, and IgG4 antibodies.

### CD209 Compounds

CD209 compounds may include full-length protein, recombinant proteins containing CD209 sequences, and CD209 protein fragments. Recombinant proteins and protein fragments may contain CD209 surface epitopes.

### Pharmaceutical Formulations

Pharmaceutical formulations may contain at least one or a combination of compounds able to bind CD209 or a CD209 compound or antibody able to affect neutrophil movement in a patient or neutrophil numbers in a body region.

In specific embodiments, the formulation may be tailored for its intended use. For example, the formulation may be suitable for administration via oral administration, topical administration, injection or inhalation. An inhalable formulation may be suitable for use in a nebulizer. Topical formulations may include any suitable cream, ointment, emollient, gel, foam, or transdermal patch as a carrier. An oral formulation may include pills, tablets, capsules, liquids, syrups, and food or drink supplements, which may include extended release formulations.

In each formulation, the compound or compounds may be contained in therapeutically effective amounts, which may vary depending on the intended mode of administration and the ease of access to the blood stream or site containing excess neutrophils using the selected mode of administration

Formulations of these compounds may also include a pharmaceutically acceptable carrier, in particular a carrier suitable for the intended mode of administration, or salts, buffers, or preservatives. In particular embodiments containing biological molecules, such as antibodies or other proteins, the pharmaceutically acceptable carrier may be tailored to allow the biological molecule to retain an active conformation and to avoid degradation.

In specific embodiments, where Ca²⁺ or another cation, such as a divalent cation contributes to binding of CD209 by the compound, the formulation may contain a sufficient amount of Ca²⁺ or other cation, such as a divalent cation to allow or enhance binding.

In still additional specific embodiments, the pharmaceutical formulation may contain stabilizers to prevent or decrease degradation of the compound or to otherwise render the formulation stable at temperatures able to prevent or decrease degradation of the compound. Sulfur (S)-containing compounds may, in particular, be prone to thermal degradation and may thus need stabilizers or storage at a low temperature.

Pharmaceutical formulations may include other pharmaceutically effective materials. For example, formulations may include Serum Amyloid P (SAP).

### Effects of CD209-Binding Compounds or antibodies

One feature of the present disclosure is the effect of CD209 and CD209-binding compounds or antibodies upon neutrophils. In some embodiments, CD209-binding compounds or antibodies may limit the influx of neutrophils into a certain region or tissue. This may be beneficial in efforts to limit an inflammation response encouraged by neutrophils or other damage resulting from the presence of excessive neutrophils in the body region. For example, in ARDS, neutrophils may cause undesired damage through escalation of an inflammation response. The inhibition of the influx of neutrophils into lung tissue may assist in the treatment of ARDS.

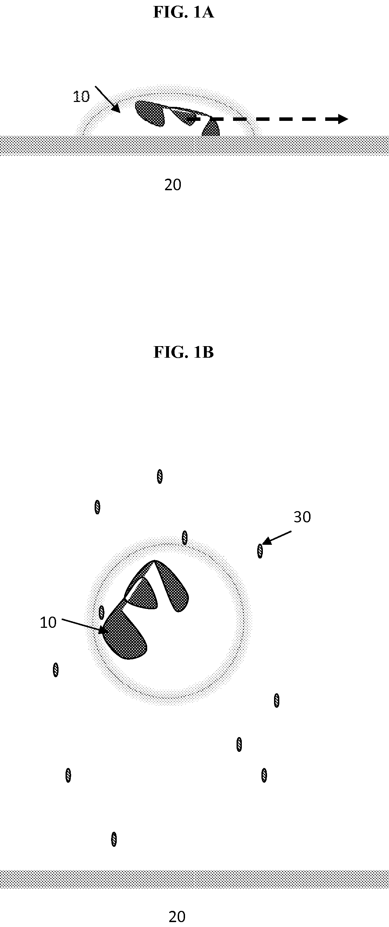

Neutrophils 10 normally bind to and move along a surface 20 as shown in FIGURE 1A. The presence of a CD209-binding compound or antibody 30 in a region suppresses neutrophil movement, as shown in FIGURE 1B.

Without limiting the present disclosure, a CD209-binding compound may or antibody exert its effects on neutrophils by affecting transmigration of neutrophils into a body region through its effects on interaction of neutrophils with endothelial cells and extracellular matrices.

### Use of CD209 and CD209-Binding Compounds

A CD209-binding compound or antibody described above, may be administered either systemically or locally to a region of the body, for example at one or more administration sites, in an amount and for a time sufficient to suppress neutrophil movement into that region or to enhance movement of neutrophils out of that region. This results in a decreased number of neutrophils in the region as compared to prior to administration of a CD209-binding compound or as compared to the number of neutrophils that would be present absent the administration of a CD209-binding compound or antibody. The decreased number of neutrophils may prevent, alleviate, or avoid one or more symptoms of complications of an acute injury or chronic or long-term disease characterized by an excess of neutrophils. In a specific embodiment, administration is systemic.

The region to which a CD209-binding compound or antibody is administered may be any body region with an unwanted number of neutrophils and the condition treated may be any acute injury or long-term or chronic disease in which an undesirable number of neutrophils are present.

In some embodiments of the present disclosure, a CD209-binding compound or antibody may be used to treat ARDS, as neutrophils appear in the lungs earlier than fibrocytes. The cells obtained from the bronchoalveolar lavage (BAL) of patients with acute respiratory distress syndrome (ARDS) show a significant increase in the number of neutrophils in the lungs compared to the cells obtained from the BAL of normal, healthy individuals. Cells obtained from the BAL of ARDS patients consist of 72% neutrophils, 25% alveolar macrophages, and 2% lymphocytes, while cells obtained from the BAL of normal, healthy individuals consist of 0% neutrophils, 91% alveolar macrophages, and 9% lymphocytes. After some time, fibrocytes can then be detected in the BAL of ARDS patients, and patients with the similar, but less severe, syndrome called acute lung injury (ALI).

Animal models of ARDS and fibrosis show different timings in the migration of innate immune cells such as neutrophils and monocytes after injury. In animal models of ARDS, the number of neutrophils in the lung increases as early as 4 hours after the injury and peaks at day 3. Macrophages and fibroblasts appear in these injured sites after 3-5 days, and the number of neutrophils remains elevated at these time points. There are neutrophils present in the injured sites as late as 2 weeks, and the numbers decline after 4 weeks. Repair cells such as fibrocytes appear at day 2, well, after neutrophils first appear, and do not reach a maximal concentration until day 8. Fibrocytes persist through day 21.

In some embodiments, a CD209-binding compound or antibody may be used to treat acute lung injury (ALI). One of the initial indications of ALI is migration of neutrophils into lung tissue. The use of a CD209-binding compound or antibody to decrease the number of neutrophils may be an effective treatment of ALI. This may occur by inhibiting the movement of neutrophils into the lungs. Additionally, this may occur by limiting an immune response, an inflammation response, or a combination of the two. Although the pathogenesis of ALI and ARDS involve both inflammation and fibrosis, the two occur at different time intervals and each stage must be treated as different disease processes. An accepted animal model of ALI involves intratracheal instillation of bleomycin, and this produces early histological events similar to those of ARDS in humans. Even though this eventually results in fibrosis, the two processes are separated by a definite time interval, and a distinction between the early inflammatory phase and the late fibrotic phase is appreciated by histological and gene expression data. Furthermore, different interventions at different time points have been shown to affect the course of the disease in independent ways. This early inflammatory stage of lung injury has been found to be mediated by an early accumulation of neutrophils.

CD209-binding compounds or antibodies may also be administered to treat long-term or chronic diseases of the lungs, such as COPD, asthma, bronchiectasis, or cystic fibrosis. As explained above, patients with COPD have a constant influx of unhealthy levels of neutrophils into their lungs. Neutrophils also play a role in asthma, especially in patients with chronic or severe asthma and asthma resistant to corticosteroids. In addition, it appears that there are increased numbers of neutrophils in non-allergic forms of asthma, such as those induced by air pollution, infection, and obesity. Therefore, in COPD and the forms of asthma where conventional therapies are not effective, treatment with a CD209-binding compound may be beneficial. Bronchiectasis appears to involve an excessive number of neutrophils damaging the airways of the lungs, causing them to widen. Therefore, treatment with a CD209-binding compound or antibody may be beneficial.

Cystic fibrosis (CF) is a single-gene disorder caused by mutations in a chloride channel (CFTR). Lung disease is a major problem for patients with CF, leading to persistent bacterial infection and exaggerated inflammatory responses with elevated numbers of neutrophils. These neutrophils appear to be responsive for the release of proteases (enzymes) that damage the lung tissues. Therefore, treatment with a CD209-binding compound or antibody may be beneficial in these situations.

In another example, the body region treated with a CD209-binding compound or antibody may be a joint and the condition may be rheumatoid arthritis, which is also characterized by excessive numbers of neutrophils.

In addition to treatments associated with the lungs and joint, the features of the present disclosure may also be beneficial in the treatment of other diseases. In some embodiments, the indication being treated may include neutrophil-induced tissue damage. By way of example, the following diseases and regions of the body may be treated within the scope of the present disclosure.

In some embodiments, the present disclosure may be used to treat traumatic brain injury. In addition to the initial insult of the traumatic event to the brain parenchyma, there is a significant amount of secondary damage produced by an influx of inflammatory cells and edema. Neutrophil accumulation in the injured brain tissue is an early event seen after traumatic brain injury. Multiple animal models have proposed a role for halting this neutrophil infiltration as a method for limiting secondary damage to injured brain tissue. One of these methods, hypothermia, has been shown to both decrease neutrophil accumulation in an animal model and produce significantly improved clinical outcomes on human patients. Therefore, controlling neutrophil influx may be an effective treatment for brain injury.

The acute phase of tissue transplant rejection may also be treated within the scope of the present disclosure. Acute tissue transplant rejection remains a significant burden in transplant medicine despite improved methods to aid pre-transplant compatibility screening, and improved post-transplant immunosuppressive drugs. This rejection is mediated by both allo-antigen primed T-cells that infiltrate the tissue and attract other inflammatory cells such as neutrophils as an effector cell to produce tissue damage, and collagen deposition that activates the complement system which also attracts and activates neutrophils. Massive early neutrophil influx into the transplanted tissue has been demonstrated in allografts undergoing acute rejection in both human patients and in animal models of cardiac, liver, kidney, lung, small bowel, and pancreas transplants. Attenuating this inflammatory neutrophilic response may prevent much of the damage done during the acute phase of rejection.

Neutrophil influx into the liver causes liver damage in alcohol-induced neutrophilic steatohepatitis and after acetaminophen overdose. Therefore, these diseases may be treated within the scope of the present disclosure.

In the kidney, neutrophil influx causes tissue damage in acute glomerulonephritis and/or renal inflammation. Acute glomerular injury has been shown to be incited by circulating immune complexes that deposit in the glomerular basement membrane. These immune complexes activate the complement cascade, which then attracts inflammatory cells that further damage the glomerular basement membrane while trying to break down these immune complexes. Neutrophils have been shown to be an important effector cell causing damage to the glomerulus in various forms of acute glomerulonephritis through production of oxidants, cytokines, and chemokines. These can include immune-complex type acute glomerulonephritides such as post-streptococcal glomerulonephritis, Goodpasture syndrome, rapidly progressive glomerulonephritis, membranoproliferative glomerulonephritis, IgA nephropathy, as well as glomerulonephritis caused by antineutrophil cytoplasmic antibody (ANCA)-associated vasculitis (AAV). The early stages of acute glomerulonephritis consist of mainly an inflammatory reaction consisting of neutrophils, with no atrophic or fibrotic changes seen in late stages of chronic glomerulonephritis. This suggests that treating these diseases early in their course with an agent that can prevent neutrophil influx and activation may ameliorate the damage caused by neutrophils during the natural history of the disease. The compounds of the present disclosure may be used to treat early stages of acute glomerulonepthritis.

The compounds or antibodies of the present disclosure may be used to treat postoperative ileus. Postoperative ileus may be described as a reduction of gastrointestinal motility following an abdominal surgery. It is a significant medical problem that causes patients prolonged discomfort, and it is one of the most common reasons for delayed discharge from the hospital after abdominal surgery. There has not been much progress towards the treatment or prevention of this consequence of surgery, other than the recent adoption of laparoscopic surgery, which has shown to significantly attenuate postoperative ileus, presumably due to a reduced amount of trauma to intra-abdominal tissues. In animal models, it has been shown that trauma to the gastrointestinal tract is evidenced on a cellular level by an early neutrophilic infiltrate. The invading neutrophils secrete many pro-inflammatory cytokines and cytotoxic substances that contribute to gastrointestinal tract dysmotility. Therefore preventing this early influx of neutrophils may lessen the amount of postoperative ileus, allowing patients to avoid unnecessary suffering, and hospitals to save a tremendous amount of resources.

Chronic and long-term intestinal diseases, such as Crohn's disease and ulcerative colitis also involve the influx of neutrophils into an intestinal tissue. The present disclosure includes periodic administration of a CD209-binding compound or antibody to reduce the number of neutrophils in such tissues and thereby to also reduce one or more symptoms of these diseases.

In some embodiments, the CD209-binding compounds or antibodies of the present disclosure may be used to treat acute pancreatitis. Acute pancreatitis remains one of the most frequent causes for hospitalization for gastrointestinal related problems, with over 250,000 admissions and costs of over $2 billion per year. Acute pancreatitis can have a wide spectrum of presentations, from mild discomfort to surgical emergencies and multi organ failure resulting in death. From clinical data, it has been demonstrated that pro-inflammatory mediators in the blood of acute pancreatitis patients correlate with the severity of that episode of acute pancreatitis. One of these mediators, PMN-elastase, which is secreted by neutrophils, was found to be one of the most useful indicators of severity. Animal models of acute pancreatitis have also exhibited an early influx of neutrophils into the pancreatic tissue, and that inhibiting this neutrophilic response by ways of neutrophil depletion and neutrophil receptor blockade have lessened the severity of the disease course of both the pancreatitis itself and associated distant organ damage. CD209-binding compounds or antibodies of the present disclosure may also be used to treat chronic pancreatitis.

In the skin, neutrophil influx causes tissue damage in Sweet's syndrome/acute febrile neutrophilic dermatosis, rheumatoid neutrophilic dermatitis, pyoderma gangrenosum, subcomeal pustular dermatosis, Behcet's syndrome, palmoplantar pustulosis, neutrophilic eccrine hidradenitis, bowel-associated dermatosis-arthritis syndrome, and synovitis-acne-pustulosis-hyperostosis osteomyelitis (SAPHO) syndrome.

Neutrophil influx may also cause tissue damage in systemic septic shock, and treatment thereof is within the scope of the present disclosure.

Gout and other crystal-induced arthropathies, which are classic inflammatory diseases where neutrophils cause a considerable amount of damage, may also be treated with formulations of the present disclosure.

Additionally, reperfusion injury, such as pressure ulcers, diabetic foot ulcers, myocardial infarction, stroke, ischemic brain injury, and ischemic bowel disease, may be treated with formulations of the present disclosure. Reperfusion injury occurs following the return of blood flow to a tissue. During the period that blood flow is restricted or stopped, the lack of oxygen (ischemia) leads to cell damage and necrosis, which when blood flow returns (reperfusion) leads to the influx of immune cells, led by neutrophils. The neutrophils are then activated by the presence of the dead and dying cells, leading to inflammation. The administration of a CD209-binding compound or antibody may inhibit the movement of neutrophils into the site of reperfusion injury, thus reducing tissue damage and preventing further influx of immune cells.

The CD209-binding compound or antibody supplied to a body or body region may include any CD209-binding compound or antibody described herein or known in the art. The body region to which a CD209-binding compound or antibody is supplied may be any region containing an unwanted number of neutrophils.

The CD209-binding compound or antibody may be supplied to the region in any suitable manner. For instance, in patients with rheumatoid arthritis, it may be injected into an administration site in an affected joint via fine needles. For a patient with danger of an abnormally strong response to a minor injury, it may be applied topically to a minor wound. For patients with ARDS, lung irritation, or any other medical problem associated with excess neutrophils in the lungs, it may be provided via inhalation, for instance using a nebulizer. For patients with systemic neutrophil problems, an injection or oral administration of a CD209-binding compound or antibody may be provided. For patients with bowel-based neutrophil indications, an ingestible form may be used. In these and other embodiments, a CD209-binding compound or antibody may be supplied systemically.

In some embodiments, involving acute neutrophil influx, a CD209-binding compound or antibody may first be administered within 30 minutes, within 45 minutes, or within 60 minutes of a severe injury that leads to an acute influx of neutrophils. In other embodiments, a CD209-binding compound or antibody may first be administered up to within 24 hours or even within 48 hours after such an injury. In many embodiments addressing acute neutrophil influx, treatment is ideally begun as soon as possible after the injury, however, not all patients or the cause of their injury are discovered until some time has passed. Treatment may be provided continuously or at intervals until the danger of neutrophil influx passes. For example, in some embodiments, treatment may be provided continuously or at intervals for at least 24 hours, at least 48 hours, at least 72 hours, at least 96 hours, or at least 120 hours. One may determine when it is appropriate to cease treatments by observing when neutrophil influx, or the symptoms of neutrophil influx, no longer occur. In embodiments in which treatments are administered at intervals, intervals may be spaced such that substantial neutrophil influx does not resume between treatments or so that a sufficient concentration of a CD209-binding compound or antibody is maintained in the body region. For example, treatments may be repeated at least every 30 minutes, at least every 60 minutes, at least every 120 minutes, or at least every 24 hours. The time between intervals may increase as time after the injury increases.

In treatment of chronic or long-term diseases, such as rheumatoid arthritis or COPD, treatment may be administered periodically at intervals sufficient to decrease the number of neutrophils in the affected body region. For example, treatment may be administered at least every 24 hours, every 48 hours, every 72 hours, every 96 hours, every 120 hours, every week, or every two weeks.

Compounds may be administered in an amount of 0.5 mg/kg of patient or target location or less, in an amount of 0.1 mg/kg of patient or target location or less, in an amount of between 0.001 and 0.05 mg/kg of patient or target location, or in an amount of between 0.01 and 0.1 mg/kg of patient or target location. Antibodies may be administered at a concentration of 0.25 mg/mL or less, at a concentration of 0.125 mg/mL or less, at a concentration of between 0.01 mg/mL and 0.25 mg/mL, or at a concentration of between 0.01 mg/mL and 0.125 mg/mL.

The amount of a CD209-binding compound or antibody administered may vary depending on the location of administration, the mode of administration, whether an acute injury or chronic or long-term disease is being addressed, the planned treatment regiment, including dosing intervals, and the severity of the injury or disease.

### CD209 Administration

As explained in the background above, some patients may benefit from increased neutrophil movement into or retention in a location. Such patients therefore may benefit from administration of CD209 decoy receptors, or removal of a CD209 ligand from that region.

### Methods of Detecting CD209 Binding Compounds and Antibodies

The present disclosure further includes methods of screening for compounds and antibodies able to affect neutrophil movement or adhesion, reduce the number of neutrophils in a body region, or treat a disease affected by neutrophil movement. Such methods involve first screening compounds and antibodies to determine if they bind to CD209, then screening any binding compounds to detect whether they also affect neutrophil movement or adhesion, reduce the number of neutrophils in a body region, or treat a disease. Binding screening may be conduced using high-throughput techniques.

### EXAMPLES

The following examples provide further details regarding certain aspects of the disclosure and are not intended to describe the complete invention.

### Example 1: Small molecule CD209 ligands inhibit neutrophil adhesion and spreading

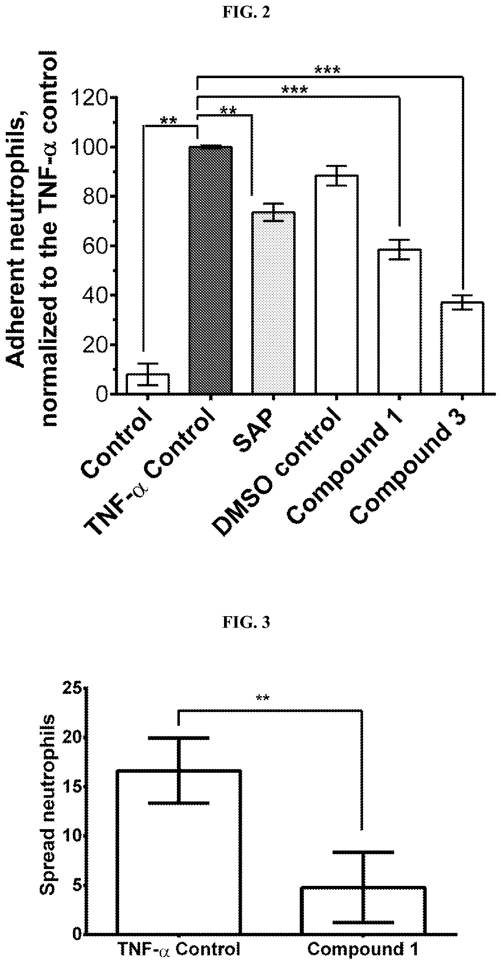

Human blood was collected into EDTA tubes (BD Bioscience, San Jose, CA) from adult volunteers who gave written consent and with specific approval from the Texas A&M University human subjects Institutional Review Board. Neutrophils were then isolated from the blood using Lympholyte-poly (Cedarlane Laboratories, Hornby, BC, Canada) following the manufacturer's protocol and resuspended in 2% bovine serum albumen (BSA) (fraction V, A3059; Sigma-Aldrich) in RPMI 1640 medium (Lonza, Allendale, NJ). 96-well tissue culture plates (flat-bottom polystyrene, BD Bioscience) were pre-coated with 20 µg/ml of human plasma fibronectin (Trevigen, Gaithersburg, MD) in phosphate buffered saline (PBS) for 30 minutes at 37°C and then blocked with 2% BSA in RPMI for 1 hour at 37°C. 50 microliters of 5 x 10⁵ cells/ml neutrophils were incubated with 3 microgram/ml (0.5 microliters of the stock solution) of compound 1 or 3 for 30 minutes at 37°C in a 1.5 ml Eppendorf tube (Eppendorf, Hauppauge, NY). When testing the effect of CD209 antibodies on neutrophil adhesion to human fibronectin, anti-human CD209 antibodies clone DCN46 (BD Biosciences, San Jose, CA), eB-h209 (eBioscience, San Jose, CA), H-200 (Santa Cruz Biotechnology, Dallas, TX), ab89186 (Abcam, Cambridge, MA), or 9E9A8 (Biolegend Inc, San Diego, CA) were incubated with neutrophils at a concentration of 1 microgram/ml (0.2 microliters of the stock solution) or the concentration indicated on the figure. Rat IgG2a was used as an isotype control for the anti-human CD209 antibody clone eB-h209. Mouse IgG2b was used as an isotype control for the anti-human CD209 antibody clone DCN46. Mouse IgG1 was used as an isotype control for the anti-human CD209 antibody clone 9E9A8. Rabbit IgG was used as an isotype control for the two rabbit anti-human CD209 antibodies clone H-200 and ab89186. The cells were then seeded into a well of a fibronectin-coated plate, and 5 microliters of 1 microgram/ml recombinant human-TNF-a (Peprotech, Rocky Hill, NJ) in 2% BSA/ RPMI was added to the neutrophils for another 30 minutes at 37°C. During this incubation period the spreading of cells was assessed using phase contrast microscopy, and the number of neutrophils that showed clear spreading on the fibronectin was scored as a percent of the total number of neutrophils in a field of view. After removing the media and washing the plates 3 times with PBS, the plates were air-dried, fixed, stained, and the number of neutrophils that remained in the wells was counted. Compound 1 (ChemBridge, San Diego, CA), and compound 3 (ChemDiv, San Diego, CA) were dissolved in demethyl sulfoxide (DMSO) (Sigma-Aldrich) to a final concentration of 200 micrograms/ml. Serum Amyloid P (SAP) was purchased from EMD Millipore (Billerica, MA) and is known to inhibit neutrophil migration and adhesion. Before use, SAP was buffer exchanged into 20 mM sodium phosphate, pH 7.4 using a 10 KDa spin column (EMD Millipore) and then diluted to a concentration of 1 milligram/ml. In the neutrophil adhesion assays, some cells were incubated with 10 microgram/ml of SAP (0.5 microliters of the stock solution). Compared to the no-TNF-α control, TNF-α increased neutrophil adhesion (FIGURE 2). Compared to the TNF-α control, DMSO did not reduce adhesion, while SAP, compound 1, and compound 3 all significantly reduced neutrophil adhesion (FIGURE 2). The results described in FIGURE 2 indicate that compounds 1 and 3 reduce human neutrophil adhesion. Compared to the TNF-α control, compound 1 also reduced neutrophil spreading (FIGURE 3).

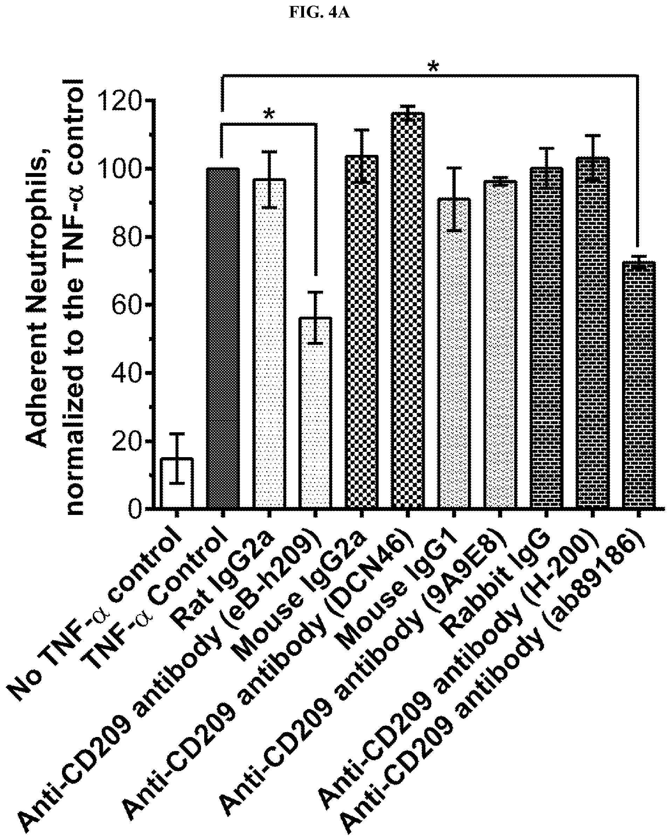

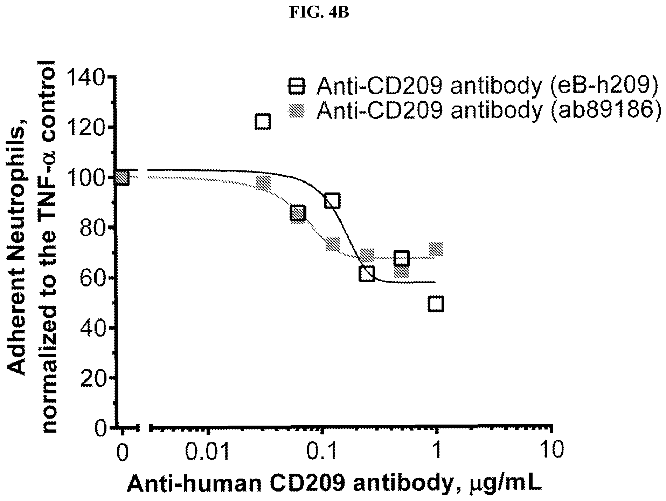

To determine if anti-human CD209 antibodies could regulate human neutrophil adhesion to human fibronectin, human neutrophils were treated with different anti-CD209 antibodies. The anti-CD209 antibody clone eB-h209 significantly decreased neutrophil adhesion to human fibronectin as compared to the no antibody TNF-α control (FIGURE 4A). More specifically, the anti-human CD209 antibody clone eB-h209 reduced neutrophil adhesion at a concentration of 0.25 micograms/ml and above (FIGURE 4B). Compared to the no antibody TNF-α control, anti-human CD209 antibody clone ab89186 significantly decreased human neutrophil adhesion to human fibronectin (FIGURE 4A). More specifically, the anti-human CD209 antibody clone ab89186 reduced neutrophil adhesion at a concentration of 0.125 micograms/ml and above (FIGURE 4B). Compared to the no antibody TNF-α control and the appropriate isotype controls, anti-CD209 antibodies clone 9E9A8, H-200, and DCN46 had no detectable effect on human neutrophil adhesion to human fibronectin (FIGURE 4A).

### Example 2: A small molecule CD209 ligand inhibits neutrophil accumulation in mice treated with bleomycin

4-week-old 20 g C57/BL6 mice (Jackson, Bar Harbor, ME.) were treated with an oropharyngeal aspiration of 50 microliters of 0.9% saline or 3 U/kg bleomycin (Calbiochem, EMD Millipore Copr. Billerica, MA.) in 50 microliters of 0.9% saline as described previously Lakatos, HF. et al., Oropharyngeal aspiration of a silica suspension produces a superior model of silicosis in the mouse when compared to intratracheal instillation, Experimental Lung Reasearch 32:181-199 (2006). The successful aspiration of bleomycin into the lungs was confirmed by listening for the crackling noise heard after the aspiration. 24 hours following bleomycin aspiration (day 1), mice were treated daily with intraperitoneal injections of 50 microliters of PBS containing 2 microliters of 1 mg/ml of compound 1 (ChemBridge Corporation, San Diego, CA) (0.1 mg/kg) dissolved in DMSO (SigmaAldrich, St. Louis, MO) or an equal volume of PBS containing 2 microliters of DMSO. All animals were used in accordance with rules published by the National Institutes of Health and with a protocol approved by the Texas A&M University Institutional Animal Care and Use Committee. Mice were weighed daily, and euthanized using CO₂ following NIH guidelines at day 3 after bleomycin aspiration. There were no injections on day 3. After euthanasia, the lungs were perfused with 300 microliters of phosphate buffered saline (PBS), pH 7.4, three times to collect cells by bronchoalveolar lavage (BAL) as described previously Lakatos, HF. et al., Oropharyngeal aspiration of a silica suspension produces a superior model of silicosis in the mouse when compared to intratracheal instillation, Experimental Lung Reasearch 32:181-199 (2006). The cells from the first BAL collection were collected by centrifugation at 500×g for 10 minutes. The BAL pellets were resuspended in the secondary and tertiary BAL fluid and the combined cells were collected by centrifugation at 500×g for 5 minutes. The cells were resuspended in 500 microliters of 4% (w/v) BSA-PBS and counted with a hemacytometer. 100 microliters of diluted cells were then aliquoted into cytospin funnels and were then spun onto glass slides (Superfrost plus white slides, VWR) at 400×g for 5 minutes using a cytospin centrifuge (Shandon, Cheshire, England). These cells were then air dried, and stained with anti-mouse Ly6G (Biolegend) to detect neutrophils, anti-mouse CD11b (Biolegend) to detect granulocytes, monocytes, and macrophages, anti-mouse CD11c (MBL International) to detect alveolar macrophages and dendritic cells, or anti-mouse CD45 (Biolegend) to detect leukocytes. For all slides, non-specific binding was blocked by incubation in PBS containing 4% BSA for 60 minutes. Slides were then incubated with 5 microgram/ml primary antibodies in 4% BSA-PBS for 60 minutes. Primary antibodies were detected with biotinylated mouse anti-rat antibody (Jackson ImmunoResearch, West Grove, PA). All secondary antibodies were used at 2.5 microgram/ml in 4% BSA-PBS for 30 minutes. Biotinylated antibodies were detected by a 1/500 dilution of ExtrAvidin alkaline phosphatase (Vector Laboratories, Burlingame, CA) in 4% BSA-PBS for 30 minutes. Staining was developed with the Vector Red Alkaline Phosphatase Kit (Vector Laboratories) for 4 minutes following the manufacturer's protocol. Slides were then counterstained for 50 seconds with Gill's hematoxylin #3 (Sigma-Aldrich, Saint Louis, MO) diluted 1:5 with water, rinsed in water, and then treated with Scott's tap water substitute (20 g/L MgSO₄·7H₂O; 2 g/L NaHCO₃) for 30 seconds. Slides were then dehydrated through 95%, and 100% ethanol, cleared with xylene, and mounted with VectaMount (Vector Laboratories). All procedures were performed at room temperature. After collection of BAL, mouse lungs were inflated with prewarmed OCT (VWR, Radnor, PA) and then embedded in OCT, frozen on dry ice, and then stored at -80°C. 6-micron cryosections of mouse lungs were mounted on Superfrost Plus microscope slides (VWR). Sections were fixed in acetone for 20 min at room temperature. Nonspecific binding was then blocked by incubation in 4% BSA (fraction V, globulin free; Sigma-Aldrich) in PBS for 60 min. Slides were then incubated with 5 microgram/ml primary antibodies in PBS containing 4% BSA for 60 min. Mouse lung sections were stained for CD11b (Biolegend) and CD11c (MBL International), Ly6G (Biolegend), CD45 (Biolegend), CD206 (Biolegend), or the appropriate isotype control (Biolegend). Slides were then washed in three changes of PBS over 30 minutes and incubated for 30 minutes with 2 microgram/ml /ml biotinylated donkey anti-rat IgG (Jackson ImmunoResearch Laboratories, Inc., West Grove, PA). After washing, the biotinylated antibodies were detected by streptavidin-alkaline phosphatase kit (Vector Laboratories) following the manufacturer's protocol.

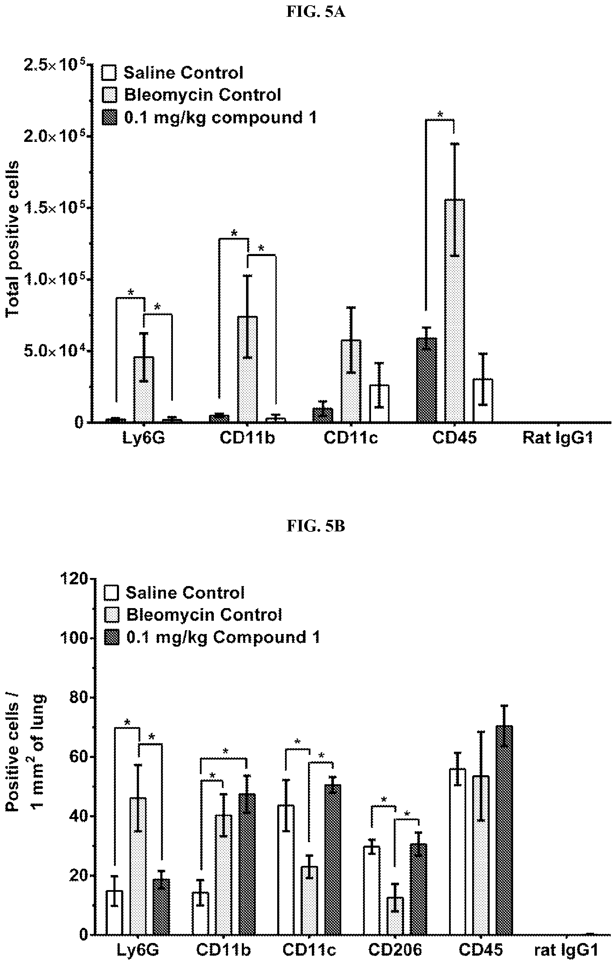

In BAL of mice, bleomycin significantly increased the number of Ly6G-positive neutrophils, CD11b-positive inflammatory macrophages, and CD45-positive cells (FIGURE 4A). After bleomycin treatment, injections of compound 1 in mice caused a decrease in the numbers of Ly6G-positive neutrophils and a decrease in the number of CD11b-positive inflammatory macrophages in the BAL, compared to injections of the drug vehicle alone (FIGURE 5A). Examining the lungs of mice after removal of weakly-adherent cells by BAL, bleomycin caused a significant increase in Ly6G-positive neutrophils and CD11b-positive inflammatory macrophages, and caused a decrease in CD11c-positive macrophages and CD206-positive cells (FIGURE 5B). For mice treated with bleomycin, compared to injections of the drug vehicle alone, injections of compound 1 caused a significant decrease in the number of Ly6G-positive neutrophils and caused a significant increase in CD11c-positive macrophages and CD206-positive cells (FIGURE 5B). Compound 1 had no effect on CD11b-positive inflammatory macrophages in the lung sections following bleomycin treatment at day 3 (FIGURE 5B). No significant changes were observed in the number of CD45 positive cells in the lung section of mice following bleomycin treatment at days 3 (FIGURE 5B).

Although only exemplary embodiments of the invention are specifically described above, it will be appreciated that modifications and variations of these examples are possible without departing from the spirit and intended scope of the invention. For example, although the effects of CD209-binding compounds or antibodies on neutrophils are specifically described herein, similar effects may be seen on other granulocytes, such as eosinophils and basophils, and may confer similar benefits. For instance, numeric values expressed herein will be understood to include minor variations and thus all embodiments relate to "about" or "approximately" the expressed numeric value unless context makes clear that a precise number is intended.

## Claims

1. A CD209-binding compound or antibody for use in a method of treating an acute injury or a chronic or long-term disease **characterized by** an excess of neutrophils in a body region by reducing the number of neutrophils in said body region, wherein the compound or antibody is administered within 24 hours, within 48 hours, or within 72 hours after an event triggering neutrophil entry into the body region.

2. The compound or antibody for use according to Claim 1, wherein the compound is Compound 1

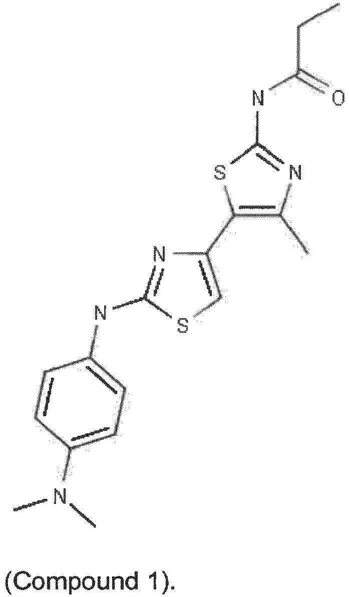

3. The compound or antibody for use of Claim 1, wherein the compound is a compound of formula I 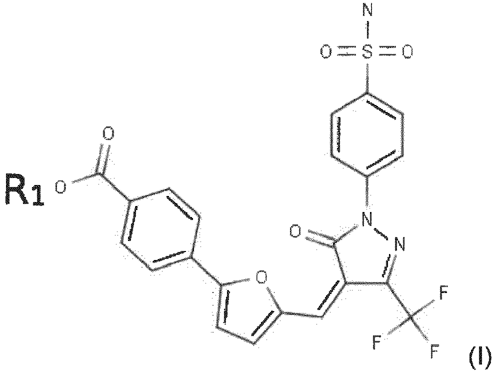 in which
R₁ is an aryl group, an alkyl group, a substituent with both aryl and alkyl components, or H.

4. The compound or antibody for use of Claim 3, wherein the compound is Compound 2 or Compound 3 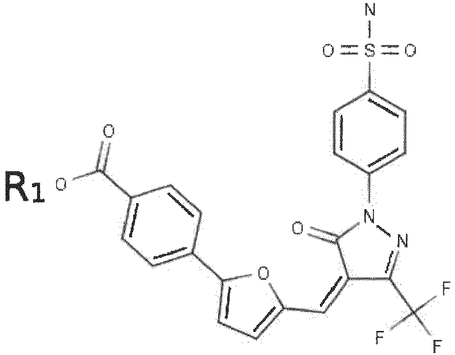 (Compound 2, wherein R₁ is a propyl group)
(Compound 3, wherein R₁ is a methyl group).

5. The compound or antibody for use of Claim 1, wherein the compound is a compound of formula II 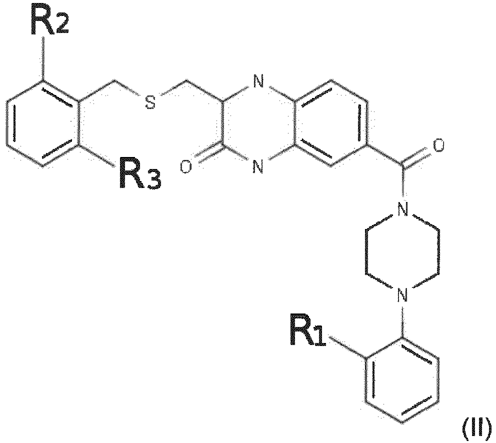 wherein
R₁ is an aryl group, an alkyl group, a substituent with both aryl and alkyl components, or H, and
R₂ and R₃ both are a monovalent halide, an aryl, an alkyl, a substituent with both aryl or alkyl components, or H.

6. The compound or antibody for use of Claim 1, wherein the compound is a compound of formula III 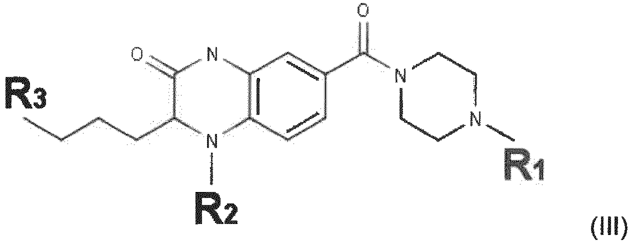 wherein
R₁, R₂, and R₃ each are an alkyl group, an aryl group, a substituent with both aryl and alkyl components, or H.

7. The compound or antibody for use of Claim 6, wherein R₁ and R₃ both are an aryl group.

8. The compound or antibody for use of Claim 1, wherein the compound is a compound of formula IV 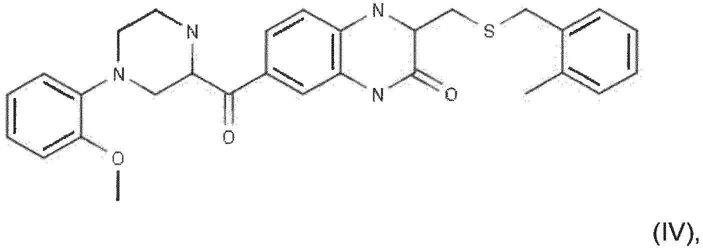 or a compound of formula V

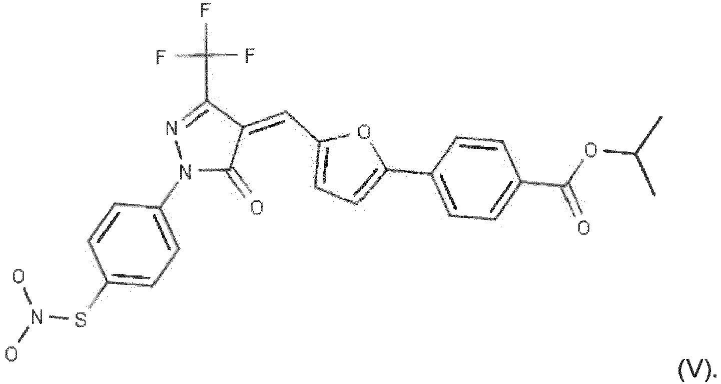

9. The compound or antibody for use of Claim 1, wherein the compound is a compound of formula VI 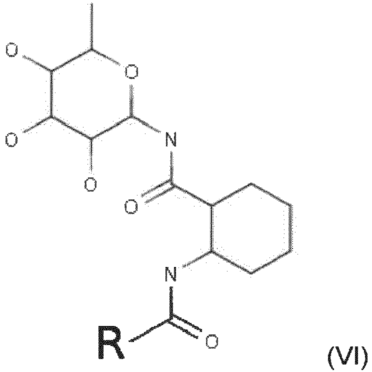 wherein
R is an aryl group, an alkyl group, a substituent with both aryl and alkyl components, or H.

10. The compound or antibody for use of Claim 1, wherein the antibody is an antihuman CD209 antibody.

11. The compound or antibody for use according to Claim 1, wherein the body region is the lungs and the compound or antibody is administered by inhalation or by nebulizer.

12. The compound or antibody for use according to Claim 1, wherein the compound or antibody is administered by injection, topical administration, oral administration, or systemic administration.

## Patentansprüche

1. CD209-bindende(r) Verbindung oder Antikörper zur Verwendung in einem Verfahren zur Behandlung einer akuten Verletzung oder einer chronischen oder langfristigen Krankheit, die durch einen Überschuß neutrophiler Granulozyten in einer Körperregion **gekennzeichnet ist, durch** Reduzierung der Anzahl neutrophiler Granulozyten in dieser Körperregion, wobei die Verbindung oder der Antikörper innerhalb von 24 Stunden, innerhalb von 48 Stunden oder innerhalb von 72 Stunden nach einem Ereignis, das den Eintritt neutrophiler Granulozyten in die Körperregion auslöst, verabreicht wird.

2. Verbindung oder Antikörper zur Verwendung nach Anspruch 1, wobei die Verbindung Verbindung 1 ist

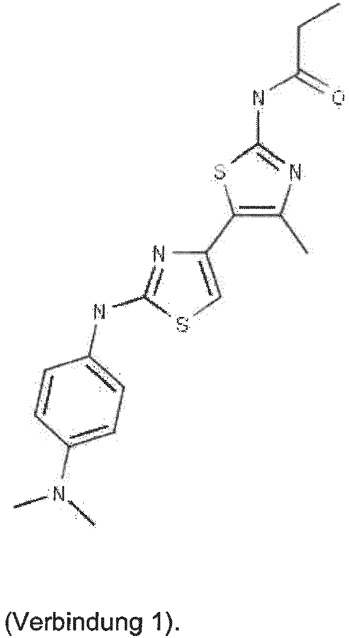

3. Verbindung oder Antikörper zur Verwendung nach Anspruch 1, wobei die Verbindung eine Verbindung nach Formel I ist 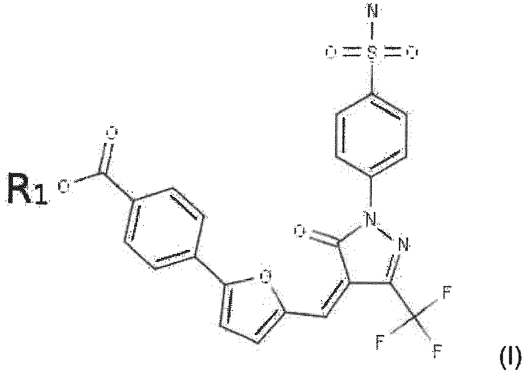 in der
R₁ eine Arylgruppe, eine Alkylgruppe, ein Substituent mit sowohl Aryl- als auch Alkylbestandteilen oder H ist.

4. Verbindung oder Antikörper zur Verwendung nach Anspruch 3, wobei die Verbindung Verbindung 2 oder Verbindung 3 ist 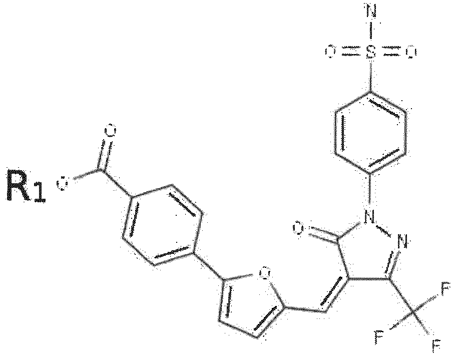 (Verbindung 2, wobei R₁ eine Propylgruppe ist)
(Verbindung 3, wobei R₁ eine Methylgruppe ist).

5. Verbindung oder Antikörper zur Verwendung nach Anspruch 1, wobei die Verbindung eine Verbindung nach Formel II ist 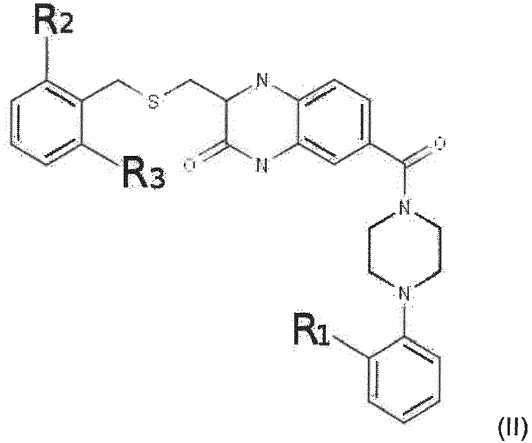 wobei
R₁ eine Arylgruppe, eine Alkylgruppe, ein Substituent mit sowohl Aryl- als auch Alkylbestandteilen oder H ist, und
R₂ und R₃ beide ein monovalentes Halid, ein Aryl, ein Alkyl, ein Substituent mit sowohl Aryl- als auch Alkylkomponenten oder H sind.

6. Verbindung oder Antikörper zur Verwendung nach Anspruch 1, wobei die Verbindung eine Verbindung nach Formel III ist 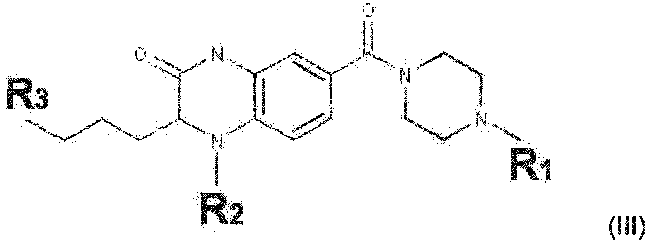 wobei
R₁, R₂ und R₃ jeweils eine Alkylgruppe, eine Arylgruppe, ein Substituent mit sowohl Aryl- als auch Alkylkomponenten oder H sind.

7. Verbindung oder Antikörper zur Verwendung nach Anspruch 6, wobei R₁ und R₃ beide eine Arylgruppe sind.

8. Verbindung oder Antikörper zur Verwendung nach Anspruch 1, wobei die Verbindung eine Verbindung nach Formel IV 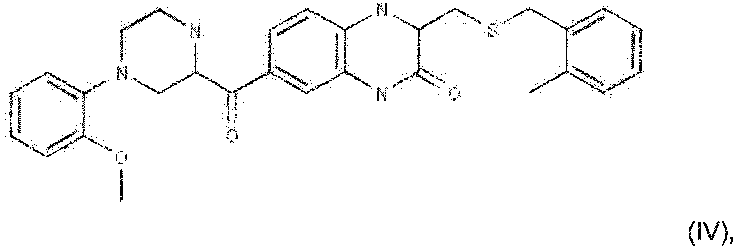 oder eine Verbindung nach Formel V ist

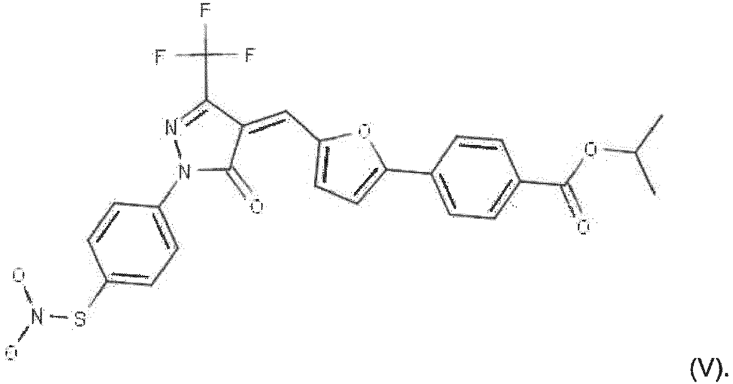

9. Verbindung oder Antikörper zur Verwendung nach Anspruch 1, wobei die Verbindung eine Verbindung nach Formel VI ist 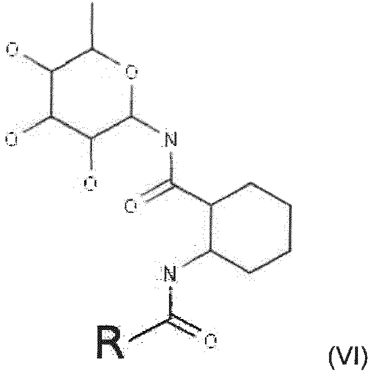 wobei
R eine Arylgruppe, eine Alkylgruppe, ein Substituent mit sowohl Aryl- als auch Alkylbestandteilen oder H ist.

10. Verbindung oder Antikörper zur Verwendung nach Anspruch 1, wobei der Antikörper ein anti-humanes CD209-Antikörper ist.

11. Verbindung oder Antikörper zur Verwendung nach Anspruch 1, wobei die Körperregion die Lunge ist und die Verbindung oder der Antikörper durch Inhalation oder durch einen Vernebler verabreicht wird.

12. Verbindung oder Antikörper zur Verwendung nach Anspruch 1, wobei die Verbindung oder der Antikörper durch Injektion, topische Verabreichung, orale Verabreichung oder systemische Verabreichung verabreicht wird.

## Revendications

1. Composé ou anticorps liant CD209, à utiliser dans un procédé de traitement d'une grave blessure ou d'une maladie chronique ou durable **caractérisés par** un excès de neutrophiles dans une région de l'organisme en réduisant le nombre de neutrophiles dans cette région de l'organisme, où le composé ou l'anticorps est administré dans les 24 heures, dans les 48 heures, ou dans les 72 heures après un événement déclenchant l'entrée des neutrophiles dans la région de l'organisme.

2. Composé ou anticorps à utiliser selon la revendication 1, dans lequel le composé est le composé 1 :

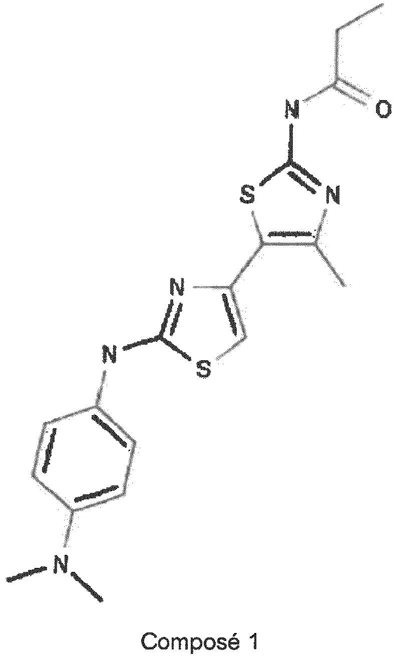

3. Composé ou anticorps à utiliser selon la revendication 1, dans lequel le composé est un composé de formule I : 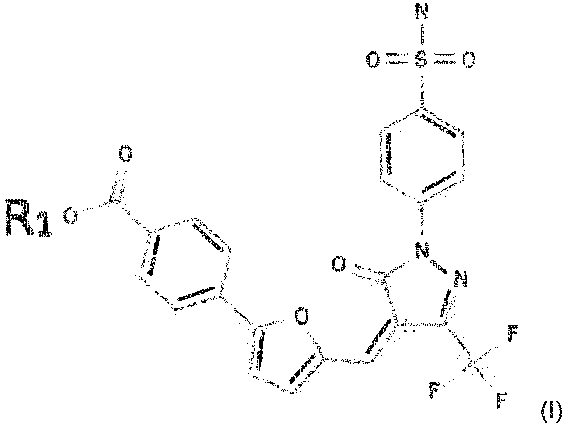 dans laquelle
R₁ est un groupe aryle, un groupe alkyle, un substituant avec à la fois des composants aryle et alkyle, ou H.

4. Composé ou anticorps à utiliser selon la revendication 3, dans lequel le composé est le composé 2 ou le composé 3 : 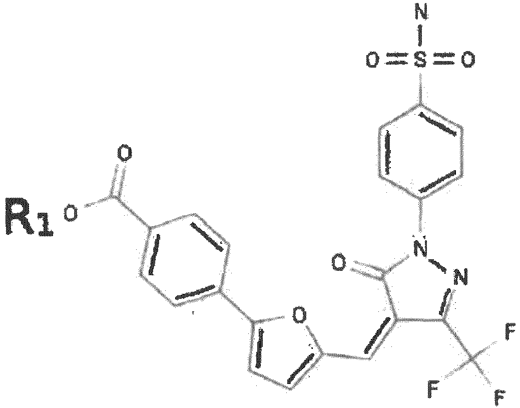 (composé 2, dans lequel R₁ est un groupe propyle)
(composé 3, dans lequel R₁ est un groupe méthyle).

5. Composé ou anticorps à utiliser selon la revendication 1, dans lequel le composé est un composé de formule II : 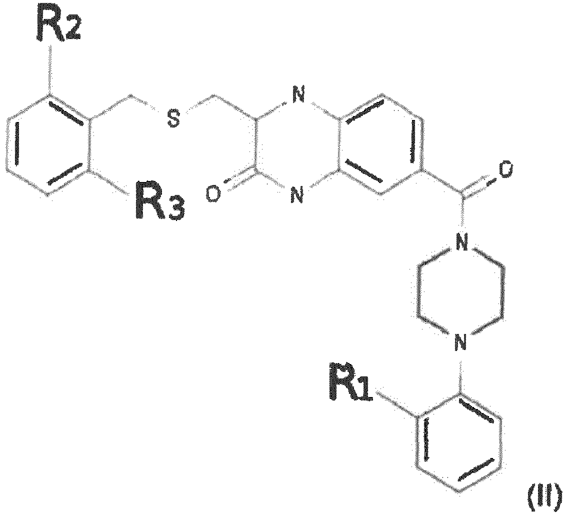 dans lequel
R₁ est un groupe aryle, un groupe alkyle, un substituant avec à la fois des composants aryle et alkyle, ou H, et
R₂ et R₃ sont un halogénure monovalent, un aryle, un alkyle, un substituant avec à la fois des composants aryle ou alkyle, ou H.

6. Composé ou anticorps à utiliser selon la revendication 1, dans lequel le composé est un composé de formule III : 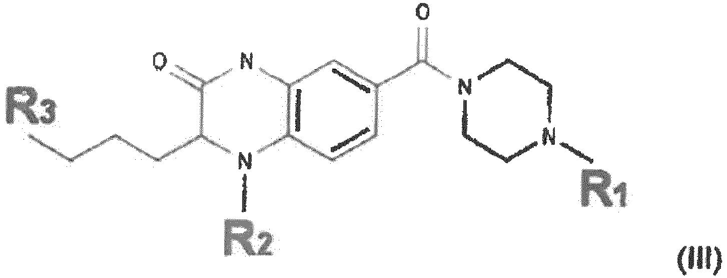 dans lequel
chaque R₁, R₂ et R₃ sont un groupe alkyle, un groupe aryle, un substituant avec à la fois des composants aryle et alkyle, ou H.

7. Composé ou anticorps à utiliser selon la revendication 6, dans lequel R₁ et R₃ sont tous deux un groupe aryle.

8. Composé ou anticorps à utiliser selon la revendication 1, dans lequel le composé est un composé de formule IV : 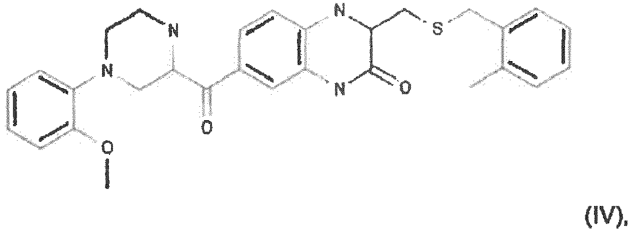 ou un composé de formule V :

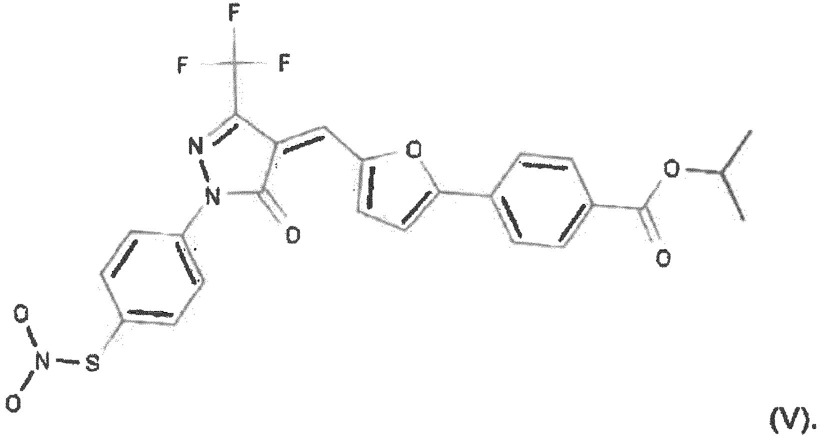

9. Composé ou anticorps à utiliser selon la revendication 1, dans lequel le composé est un composé de formule VI : 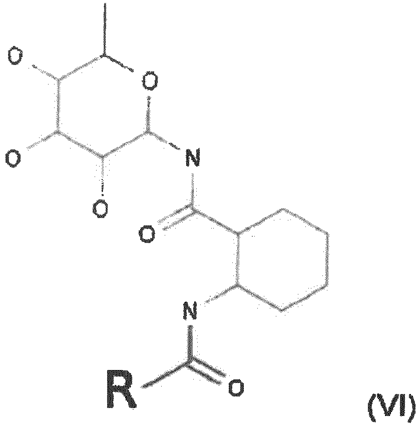 dans lequel R est un groupe aryle, un groupe alkyle, un substituant avec à la fois des composants aryle et alkyle, ou H.

10. Composé ou anticorps à utiliser selon la revendication 1, dans lequel l'anticorps est un anticorps anti-CD209 humain.

11. Composé ou anticorps à utiliser selon la revendication 1, dans lequel la région de l'organisme est les poumons et le composé ou anticorps est administré par inhalation ou au moyen d'un nébuliseur.

12. Composé ou anticorps à utiliser selon la revendication 1, dans lequel le composé ou anticorps est administré par injection, administration topique, administration orale ou administration systémique.
